# FASCICULE DE BREVET EUROPEEN

(11) **EP 1 268 470 B1**
(45) Date de publication et mention de la délivrance du brevet: **27.02.2008**
(21) Numéro de dépôt: 01921476.6
(22) Date de dépôt: 04.04.2001
(51) Int. Cl.: C07D 405/12, C07D 471/04, A61K 31/4709, A61P 33/06

(54) **MOLECULES DUALES CONTENANT UN DERIVE PEROXYDIQUE, LEUR SYNTHESE ET LEURS APPLICATIONS THERAPEUTIQUES**
DUALE MOLEKÜLE ENTHALTEND EIN PEROXYDDERIVAT DEREN SYNTHESE UND DEREN VERWENDUNG ALS HEILMITTEL
DUAL MOLECULES CONTAINING A PEROXIDE DERIVATIVE, SYNTHESIS AND THERAPEUTIC APPLICATIONS THEREOF

(30) Priorité: 06.04.2000 FR 0004422
(43) Date de publication de la demande: 02.01.2003
(73) Titulaire: CENTRE NATIONAL DE LA RECHERCHE SCIENTIFIQUE, 75974 Paris (FR)
(72) Inventeur: MEUNIER, Bernard, F-31320 Castanet (FR); ROBERT, Anne, F-31000 Toulouse (FR); DECHY-CABARET, Odile, F-31000 Toulouse (FR); BENOIT-VICAL, F. Résidence "Le soleil de laMer", F-24350 Palavas les Flots (FR)
(74) Mandataire: Peaucelle, Chantal
(86) Numéro de dépôt international: PCT/FR2001/001013
(87) Numéro de publication internationale: WO 2001/077105

(56) Documents cités:
- WO-A-97/18193
- G.E. BASS ET AL.: "MECHANISM OF ANTIMALARIAL ACTIVITY OF CHLOROQUINE ANALOGS" JOURNAL OF MEDICINAL CHEMISTRY., vol. 14, no. 4, 1971, pages 275-283, XP002171170 AMERICAN CHEMICAL SOCIETY. WASHINGTON., US ISSN: 0022-2623

## Description

L'invention a pour objet des molécules duales contenant un dérivé peroxydique, possédant notamment une activité antipaludique, leur synthèse et leurs applications thérapeutiques.

Le paludisme est l'une des premières causes infectieuses de mortalité au monde et touche chaque année 100 à 200 millions de personnes. La forte recrudescence de la maladie observée depuis quelques années est due à plusieurs facteurs, dont :
- les vecteurs, à savoir les anophèles, qui deviennent résistants aux insecticides classiques et bon marché comme le DDT, (abréviation de trichloro-1,1,1-bis (p-chlorophényl) - 2,2 éthane) ;
- l'augmentation de la population dans les zones à risque, et, principalement,
- la résistance de nombreuses souches de *Plasmodium falciparum,* parasite responsable des formes mortelles de la maladie, aux médicaments classiquement utilisés, tels que la chloroquine et la méfloquine. La découverte de l'artémisinine (1, 2) puissant antipaludique extrait de *Artemisia anmia,* a attiré l'attention vers des molécules présentant, comme l'artémisinine, une fonction endoperoxyde (3, 4). L'artémisinine et certains de ses dérivés hémi-synthétiques, tels que l'artéméther et l'artésunate, se sont révélés très actifs sur les souches résistantes de *P. falciparum.* Cependant, le coût élevé de ces composés d'origine naturelle et les aléas d'approvisionnement représentent des inconvénients majeurs. On mesurera donc l'intérêt de composés antipaludiques de synthèse, qui seraient accessibles à bas prix, et présenteraient un mécanisme d'action semblable à celui de l'artémisinine, à savoir un effet alkylant sur l'hème et/ou des protéines parasitaires.

La recherche de tels composés par les inventeurs les a amenés à développer une nouvelle stratégie de synthèse basée sur l'utilisation de composés susceptibles à la fois d'être accumulés efficacement dans le parasite et d'exercer un effet du type de celui de l'artémisine.

Les inventeurs ont constaté qu'en formant un adduit covalent entre un composé doté de propriétés antipaludiques et un dérivé de type peroxydique, on disposait de produits de couplage avec, de manière surprenante, un effet synergique entre la faculté de pénétration et l'activité des constituants respectifs sur des souches chloroquino-résistantes, et de manière générale une grande efficacité pour un large spectre de parasites.

L'invention vise donc des molécules duales se présentant sous forme de produits de couplage, possédant notamment une activité antipaludique, en particulier sur *P. falciparum.*

Elle a également pour but de fournir un procédé de synthèse de telles molécules, comportant un nombre limité d'étapes, mettant en jeu des produits de faible coût, et donc aisément exploitable à l'échelle industrielle.

L'invention vise en outre les applications biologiques de ces molécules et tout particulièrement la mise à profit de leurs propriétés antipaludiques pour l'élaboration de médicaments.

Les molécules duales de l'invention sont caractérisées en ce qu'il s'agit de produits de couplage répondant à la formule (I) 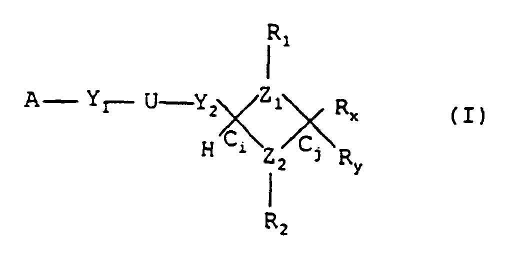 dans laquelle
- A représente un résidu de molécule à activité antipaludique,
- Y₁ et Y₂, identiques ou différents, représentent une chaîne alkylène en C1 à C5, linéaire ou ramifiée, contenant le cas échéant un ou plusieurs radicaux amine, amide, sulfonamide, carboxyle, hydroxyle, éther ou thioéther, cette chaîne alkylène en C1 à C5 étant le cas échéant substituée par un radical alkyle en C1 à C5, l'un de Y₁ ou Y₂ pouvant être absent,
- U est une fonction amine, amide, sulfonamide, carboxyle, éther ou thioéther, cette fonction assurant le lien entre Y₁ et Y₂,
- Z₁ et Z₂, identiques ou différents, représentent un radical arylène ou alkylène linéaire, ramifié ou cyclique, saturé ou insaturé, l'un de Z₁ ou Z₂ pouvant être absent, ou l'ensemble Z₁ + Z₂ représente une structure polycyclique incluant les carbones de jonction Ci et Cj,
- R₁ et R₂ identiques ou différents représentent un atome d'hydrogène ou un groupe fonctionnel capable d'augmenter l'hydrosolubilité de la molécule duale, avantageusement choisi parmi -COOH, -OH, -N(Rₐ, R_{b}) avec Rₐ et R_{b}, identiques ou différents, représentant un atome d'hydrogène ou un radical alkyle en C1 à C5,
- Rₓ et R_{y} forment un peroxyde cyclique de 4 à 8 chaînons, pouvant comporter 1 ou 2 atomes d'oxygène supplémentaires dans la structure cyclique, Cj étant l'un des sommets de ce peroxyde cyclique, ou
- Rₓ ou R_{y} est un peroxyde cyclique de 4 à 8 chaînons, pouvant comporter 1 ou 2 atomes d'oxygène supplémentaires dans la structure cyclique, et un ou plusieurs substituants R₃ identiques ou différents, occupant des positions distinctes quelconque sur le cycle, l'un au moins représentant un atome d'halogène, un groupe -OH, un groupe -CF₃, un radical aryle, un radical alkyle ou alkoxy en C1 à C5, un groupe -NO₂, le ou les autres substituants présentant l'une de ces significations ou un atome d'hydrogène, les sommets carbonés du peroxyde cyclique pouvant être le cas échéant substitués par un ou plusieurs substituants tels que définis pour R₃, deux substituants adjacents pouvant former une structure cyclique à 5 ou 6 chaînons , saturée ou insaturée, le cas échéant substituée par un ou plusieurs substituants R₃ en position quelconque, l'autre substituant Rₓ ou R_{y} pouvant être R₃,
et leurs sels d'addition avec des acides pharmacologiquement acceptables.

De manière avantageuse, le résidu A draîne à l'intérieur du parasite le composé couplé, qui exerce alors un effet alkylant sur l'hème et /ou les protéines parasitaires.

Dans une famille préférée de dérivés selon l'invention, A représente un hétérocycle azoté choisi parmi une aminoquinoléine de formule (II) ou une 1,5-naphtyridine de formule (III) suivantes 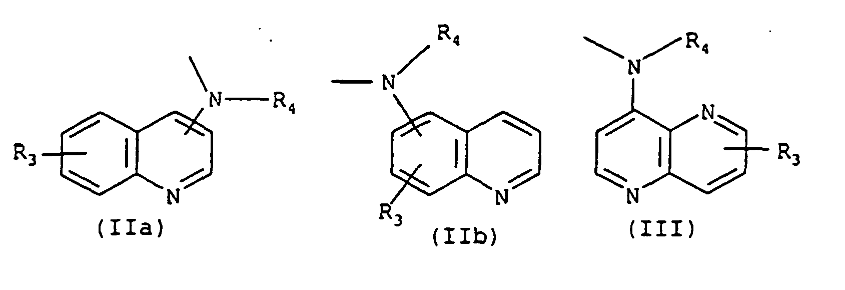 dans lesquelles
- R₃ représente, un ou plusieurs substituants identiques ou différents, occupant des positions distinctes, l'un au moins représentant un atome d'halogène, un groupe -OH, un groupe -CF₃, un radical aryle, un radical alkyle ou alkoxy en C1 à C5, un groupe -NO₂, le ou les autres substituants présentant l'une de ces significations ou un atome d'hydrogène,
- R₄ représente un radical alkyle en C1 à C5, linéaire, ramifié ou cyclique, ou un atome d'hydrogène.

Dans une autre famille préférée de l'invention, A représente un radical de formule (IV)

R₅-CHOH- (IV)

dans laquelle R₅ représente un radical aryle ou un résidu hétérocyclique azoté.

Des significations préférées pour R₅ correspondent à des radicaux 9-phénanthrényle ou 4-quinoléinyle, éventuellement substitués par un ou plusieurs groupes R₃.

Dans encore une autre famille préférée, A représente un résidu phénol-2 (aminométhyl) de formule (V) 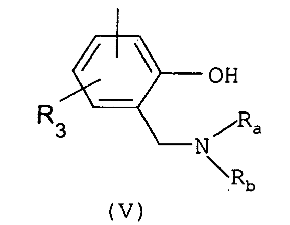 dans laquelle R₃, Rₐ et R_{b} sont tels que définis ci-dessus.

Une autre famille préférée de molécules duales selon l'invention comprend un substituant A représentant un résidu biguanide choisi parmi les dérivés de proguanil de formule (VI) 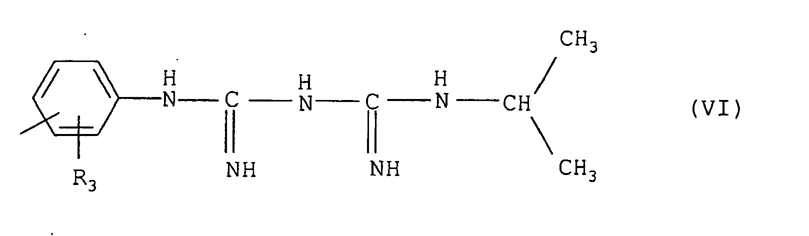 ou de cycloguanil de formule (VII) 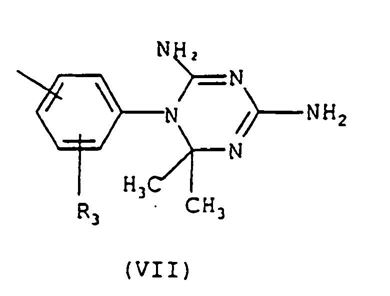 dans laquelle R₃ est tel que défini ci-dessus.

Dans une autre famille préférée, A représente un résidu de pyrimidine et plus particulièrement de pyriméthamine de formule (VM) 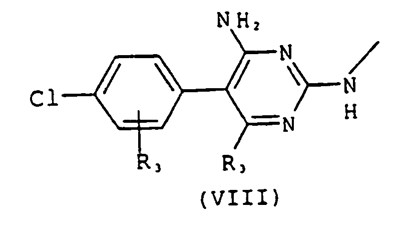 ou de formule (IX) 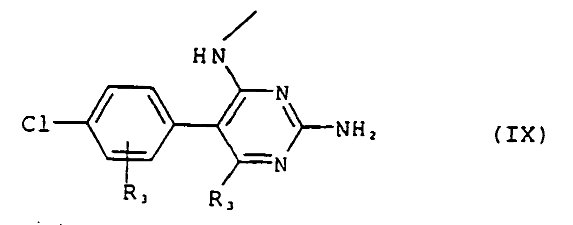 dans laquelle R₃ est tel que défini ci-dessus.

Dans encore une autre famille préférée de l'invention, A représente un résidu acridine de formule (X) 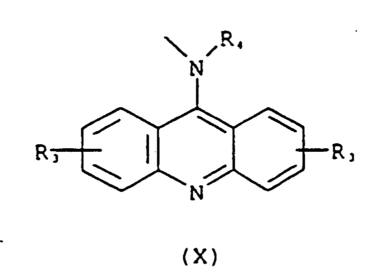 dans laquelle R₃ et R₄ sont tels que définis ci-dessus.

L'invention vise des molécules duales, telles que définies ci-dessus, en particulier correspondant aux familles préférées évoquées plus haut, et dans lesquelles Rₓ et R_{y} forment ensemble un peroxyde cyclique.

Dans des molécules duales plus spécialement préférées de ce type, Rₓ et R_{y} représentent un trioxane substitué par un ou plusieurs substituants R₃.

Dans une autre disposition préférée de l'invention, utilisée avantageusement avec la disposition qui précède, Z₁ et Z₂ représentent un radical cyclohexyle ou bis-cyclopenthyle.

Dans encore une autre disposition préférée, utilisée le cas échéant avec au moins l'une des dispositions qui précèdent, Y₁- U -Y₂ sont choisis de manière à moduler l'hydrosolubilité de la molécule afin de lui conférer une activité optimale.

L'invention vise également un procédé de synthèse des molécules définies ci-dessus.

Ce procédé comprend la réaction de dérivés réactifs de A et de dérivés peroxydiques comportant les résidus Rₓ et R_{y} de manière à former, entre ces dérivés, un bras de couplage tel que défini en rapport avec la formule (I).

Diverses voies de synthèse seront aisément accessibles à l'homme du métier en opérant selon les techniques classiques. Pour la synthèse par exemple des peroxydes, on se référera avec avantage à l'ouvrage de S. Pataï, "The Chemistry of peroxides", John Wiley and Sons Ltd, 1983.

Ainsi, pour préparer des molécules duales renfermant comme peroxyde, un trioxane, et comme dérivé A, une aminoquinoléine,
**a) la réaction d'un** composé de formule (XI) 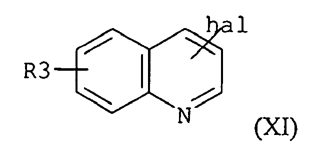 dans laquelle R₃ est tel que défini ci-dessus, et "hal" représente un atome d'halogène, avec une dérivé diaminé de formule (XII)

   R₄-NH-Y₁-U₁ (XII)

   où R₄ et Y₁ sont tels que définis ci-desus et U₁ représente un groupe -NH₂,
   ce qui conduit à l'obtention d'un composé de formule (XIII) 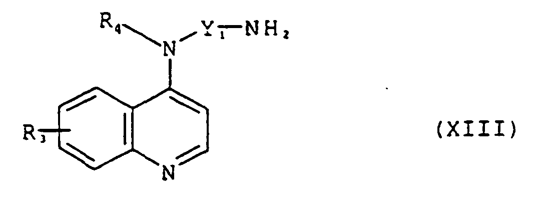 dans laquelle, R₃, R₄ et Y₁ sont tels que définis ci-dessus,
b) - l'irradiation en présence d'oxygène moléculaire et d'un photosensibilisateur, d'un dérivé de formule (XIV) à (XVII) suivante 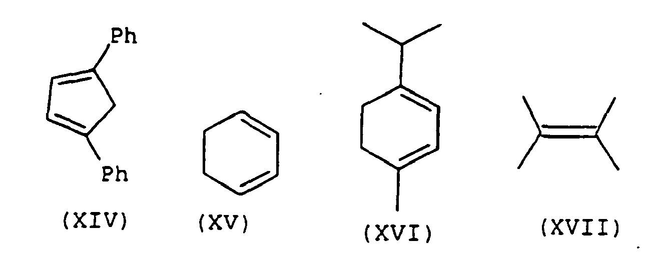 suivie de la réaction avec une dicétone, telle que la 1,4-cyclohexadione de formule (XVIII) ou la *cis*-bicyclo[3.3.0]octane-3,7-dione de formule (XIX) 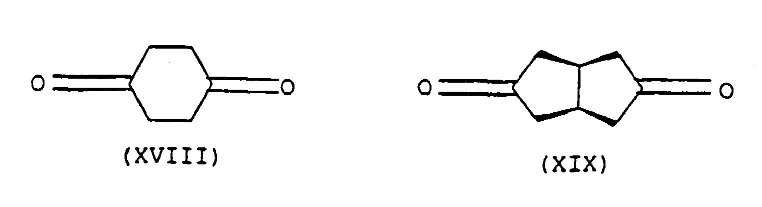 conduisent à des trioxanes fonctionnalisés par une cétone, de formule générale (XX) 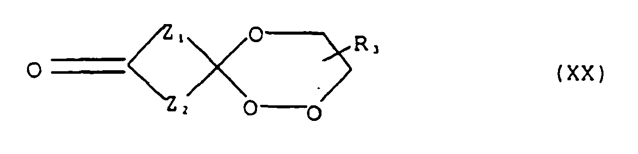 dans laquelle Z₁, Z₂ et R₃ sont tels que définis ci-dessus,
c) - le couplage du dérivé de formule (XIII) avec le trioxane de formule (XX), par amination réductrice, suivi le cas échéant d'une réaction avec un acide pharmaceutiquement acceptable, pour obtenir le produit de couplage sous forme de sel.

L'étape a) est avantageusement réalisée à une température de 80°C à 140°C et sous agitation. Le dérivé diaminé est utilisé de préférence à raison de 5 équivalents molaires. Après refroidissement, le produit obtenu est récupéré par extraction, par exemple à l'aide d'un solvant organique tel que le dichlorométhane, puis traité, si on le souhaite, aux fins de purification.

Pour réaliser l'étape b), on procède à une oxygénation photosensiblisée de l'oléfine de départ en présence d'oxygène moléculaire. Le photosensibilisateur est avantageusement un agent classique comme la tétraphénylporphyrine ou le rose de Bengale.

On fait alors réagir le peroxyde obtenu avec une dicétone, à raison de préférence de 4 à 10 équivalents molaires. La réaction est avantageusement réalisée en présence de triméthyl silyl trifluorométhane sulfonate, à température inférieure à -50°C, notamment de -70°C, pendant plusieurs heures. Le trioxane fonctionnalisé est ensuite purifié. On a recours par exemple à une chromatographie sur colonne.Un protocole voisin est utilisé pour la synthèse du trioxane précurseur des trioxaquines de formule (XIII) : le 2,3-diméthylbut-2-ène est photooxygéné dans les conditions ci-dessus, puis est mis en présence de 2 à 10 équivalents molaires d'un oxoaldéhyde, de quelques gouttes d'acide trifluoracétique et 2 équivalents molaires de N-iodosuccinimide. La réaction est réalisée à température ambiante et à l'abri de la lumière pendant plusieurs heures. Le trioxane fonctionnalisé est ensuite purifié, on a recours par exemple à une chromatographie sur colonne.

L'étape de couplage de c) entre la cétone et l'amine primaire est réalisée en présence d'un agent réducteur tel que le triacétoxyborohydrure de sodium, à température ambiante.

Ces composés sont mis en oeuvre selon un rapport molaire amine /cétone primaire d'environ 1,25, l'agent réducteur étant utilisé à raison de 1,25 équivalent/cétone. Pour obtenir le produit de couplage sous forme de sel, on procède à la protonation des azotes basiques, en ajoutant un acide pharmacologiquement acceptable. A titre d'exemple, on citera l'acide citrique, tartrique, oxalique et fumarique.

La réaction peut être réalisée avec 2 équivalents d'acide. Le produit protoné est ensuite récupéré et soumis à une ou plusieurs étapes de purification si on le souhaite.

L'étude des propriétés pharmacologiques des produits de couplage de l'invention a montré qu'ils exercent un effet antipaludique sur *P. falciparum* cultivé dans les hématies humaines.

L'obtention d'un tel effet est d'autant plus grand que les phénomènes de résistance de souches de *Plasmodium falciparum,* l'espèce mortelle, vis-à-vis des médicaments antimalariques usuels se développent et que, de plus, la protection vaccinale, pour laquelle d'importantes recherches sont effectuées, ne pourra être réalisée avant plusieurs années.

L'invention vise donc la mise à profit des propriétés de ces produits de couplage, qui de plus, présentent l'intérêt d'une grande innocuité, pour l'élaboration de compositions pharmaceutiques.

Les compositions pharmaceutiques de l'invention sont caractérisées en ce qu'elles renferment une quantité efficace d'au moins un produit de couplage tel que défini ci-dessus, en association avec un véhicule pharmaceutiquement inerte.

Ces compositions renferment le cas échéant des principes actifs d'autres médicaments. On citera notamment leur association avec toute autre molécule antipaludique (amino-quinoléine, aryl-alcool comportant une fonction amine, dérivés aminés de l'ortho-crésol, sulfones, sulfonamides, biguanides, amino-pyrimidines, amino-triazines, ou quinazolines, ainsi que les antibiotiques (tétracycline, rifampicine, gramicidine D, valinomycine et quinolones en particulier) et antifongiques présentant une activité antipaludique).

On les utilisera également avec avantage en association avec des composés facilitant leur assimilation tels que des sucres comme le glucose.

Les compositions de l'invention sont particulièrement appropriées pour le traitement du paludisme.

L'invention vise donc également l'application des produits de couplage définis plus haut pour élaborer des médicaments pour le traitement du paludisme.

Les conditionnements en vue de la vente, en particulier l'étiquetage et les notices d'emploi, et avantageusement l'emballage sont élaborés en fonction de l'application thérapeutique spécifique prévue.

Les compositions pharmaceutiques de l'invention sont administrables sous différentes formes, plus spécialement par voie orale, rectale ou injectable.

Les compositions administrées par voie orale renferment avantageusement de 40 à 300 mg de principe actif par unité de prise, de préférence de 40 à 100 mg. Elles se présentent avantageusement sous forme, notamment, de comprimés, pilules, tablettes, gélules, gouttes.

Les formes injectables renferment par unité de prise de 20 à 300 mg de principe actif, de préférence de 50 à 100 mg. Elles se présentent sous forme de solutions injectables par voie intraveineuse, sous-cutanée ou intramusculaire, élaborées à partir de solutions stériles ou stérilisables. Il peut s'agir également de suspensions ou d'émulsions.

Pour l'administration par voie rectale, on a recours à des suppositoires.

A titre indicatif, la posologie utilisable chez l'homme correspond aux doses suivantes : ainsi on administre par exemple au patient 50 à 300 mg/jour, en une ou plusieurs prises pour le traitement paludisme.

L'invention vise encore les réactifs biologiques dont les principes actifs sont constitués par les dérivés définis plus haut.

Ces réactifs peuvent être utilisés comme références ou étalons dans des études d'éventuelles activités antipaludiques.

D'autres caractéristiques et avantages de l'invention apparaîtront dans les exemples qui suivent relatifs à l'obtention de produits de couplage de quinoléines et de trioxanes, appelés "trioxaquines", et à l'étude de leur activité antiparasitaire. Les formules des composés 1 à 34 dont la synthèse est décrite dans ces exemples sont données en fin de description.

### Exemple 1 : trioxaquine 4

### Synthèse de la 7-chloro-4-[N-(2-aminoéthyl)amino]-quinoléine 1

Un mélange de 4,7-dichloroquinoléine (2,0 g, 10 mmol) et de 1,2-diaminoéthane (2,7 g, 45 mmol), est chauffé à 85 °C pendant 5h sous agitation magnétique. Après ajout de soude 1N (15 mL), le solide obtenu est extrait par de l'acétate d'éthyle (100 mL) à 50 °C. La phase organique est lavée à l'eau distillée, puis à l'aide d'une solution saturée de NaCl, puis à nouveau à l'eau distillée et enfin est séchée sur sulfate de sodium. Le solvant est évaporé et le produit obtenu est séché sous vide (1,3 g, 58 %).
RMN ¹H (250 MHz, CDCl₃) δ, ppm : 8,52 (d,³*J*_{HH} = 5,5 Hz, 1H, H2'), 7,94 (d,⁴*J*_{HH} = 2,2 Hz, 1H, H8'), 7,72 (d,³*J*_{HH} = 8,9 Hz, 1H, H5'), 7,35 (dd,³*J*_{HH} = 8,9 Hz, ⁴*J*_{HH} = 2,2 Hz, 1H, H6'), 6,40 (d,³*J*_{HH} = 5,5 Hz, 1H, H3'), 5,76 (s large, 1H, *H*N9'), 3,32 (m, 2H, *H₂*C10'), 3,11 (tr, 2H, *H₂*C11'), 1,39 (s large, *H₂*N12').
SM (DCI/NH₃⁺) *m*/*z* (%) : 221 (2), 222 (MH⁺, 100), 223 (14), 224 (33), 225 (4).

### Synthèse du trioxane fonctionnalisé par une cétone 2

Un mélange de 1,4-diphényl-'1,3-cyclopentadiène (50 mg, 0,23 mmol) et de tétraphénylporphyrine (5 mg) dans du dichlorométhane (5. mL) est irradié en présence d'oxygène moléculaire (1,15 bar) pendant 1 heure, à 5 °C, avec une lampe blanche (200 W). Le peroxyde est obtenu avec un rendement quantitatif Le peroxyde brut en solution dans le dichlorométhane est placé dans un bain à - 70 °C ; 10 équivalents molaires de 1,4-cyclohexadione (260 mg, 2,3 mmol) et 0,5 équivalent de triméthylsilyl trifluorométhane sulfonate (20 µL, 0,11 mmol) sont ajoutés et le mélange réactionnel est maintenu sous agitation à - 70 °C pendant 4 heures. La réaction est arrêtée par addition de triéthylamine (40 µL). Après retour à la température ambiante, le milieu réactionnel est lavé à l'eau distillée, séché sur sulfate de magnésium et évaporé à sec. Le trioxane fonctionnalisé 2 est purifié par chromatographie sur colonne de silice (éluant hexane/acétate d'éthyle, 80/20, v/v) (rendement : 55 %).
RMN ¹H (250 MHz,CDCl₃) δ, ppm : 7,60-7,30 (m, 10H, H-phényle), 6,35 (dd, *J*_{HH} = 1,6 et 4,0 Hz, 1H, H6), 5,26 (s large, 1H, H5), 3,31 et 3,05 (2 x d, ²*J*_{HH} = 17,0 Hz, 2 x 1H, *H₂*C8), 2,56-2,43 (m, 5H), 2,26 (m, 1H), 2,05 (m, 2H).
SM (DCI/NH₃⁺) *m*/*z* (%) : 363 (MH⁺, 24), 364 (7), 380 (MNH₄⁺, 100), 381 (27), 382 (7).

### Couplage de l'amine primaire avec la cétone par amination réductrice : obtention de la trioxaquine 3

La cétone **2** (99 mg, 0,27 mmol) et l'amine primaire 1 (76 mg, 0,34 mmol) sont mises en solution dans CH₂Cl₂ (5 mL). On ajoute le triacétoxyborohydrure de sodium (72 mg, 0,34 mmol). Le mélange est maintenu sous agitation à température ambiante pendant 18 heures. Le milieu réactionnel est ensuite lavé à l'eau distillée ; la phase organique est séchée et le solvant est évaporé à sec (rendement : 87 %).
RMN ¹H (250 MHz, CDCl₃) δ, ppm : 8,50 (2 x d, 1H, H2'), 7,95 (2 x d, 1H, H8'), 7,70 (2 x d, 1H, H5'), 7,63-7,25 (m, 11H, H6' et 10H phényle), 6,35 (m, 2H, H3' et H6), 5,99 (s large, 1H, *H*N9'), 5,17 (2 x s large, 1H, H5), 3,31 (m, 3H, *H*₂C10' et *H*C8), 3,05 (m, 3H, *H*₂C11' et *H*C8), 2,61 (m, 2H, cyclohexyle), 2,42 (m, 1H, *H*C12), 2,10-1,25 (m, 7H, 6H, cyclohexyle et *H*N12').
SM (DCI/NH₃⁺) *m*/*z* (%) : 566 (11), 568 (MH⁺, 100), 569 (38), 570 (41), 571 (12).

### Obtention du dicitrate de trioxaquine 4

La trioxaquine **3** (25 mg, 0,04 mmol) est mise en solution dans l'acétone (0,5 mL). On ajoute de l'acide citrique (17 mg, 2,0 équiv.) en solution dans l'acétone (0,5 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,59 (2 x d, 1H, H2'), 8,30 (2 x d, 1H, H5'), 7,95 (2 x d, 1H, H8'), 7,70 (m, 5H, H6' et 4H phényle), 7,50 (m, 6H, phényle), 6,73 (2 x d, 1H, H3'), 6,60 (2 x q, 1H, H6), 5,42 (2 x s large, 1H, H5), 3,71 (m, 2H, *H₂*C10'), 3,55-3,25 (m, 4H, *H₂*C11', *H*C8 et *H*C12), 3,12 (d, 1H, *H*C8), 2,76 (d, 4H, citrate), 2,65 (d, 4H, citrate), 2,10-1,50 (m, 8H, cyclohexyle).

| | |
|---|---|
| SM (ES) *m*/*z* (%) : | en mode positif 568,2 (M⁺) |
| | en mode négatif 190,9 (citrate) |

| Microanalyse élémentaire : pour C₄₆H₅₀O₁₇N₃Cl | | | |
|---|---|---|---|
| % Théor. : | C 58,01 | H 5,29 | N 4,41 |
| % Expér. : | C 57,65 | H 5,09 | N 4,49 |

### Exemple 2 : trioxaquine 7

### Synthèse de la 7-chloro-4-[N-(3-aminopropyl)amino]-quinoléine 5

Un mélange de 4,7-dichloroquinoléine (5 g, 25 mmol) et de 1,3-diaminopropane (9,3 g, 126 mmol), est chauffé au reflux de la diamine (118 °C) pendant 5 heures sous agitation magnétique. Après refroidissement, le solide obtenu est extrait par du dichlorométhane (3 x 100 mL) à reflux. La phase organique est lavée à l'eau distillée, séchée sur sulfate de sodium. La concentration de la phase dichlorométhane, puis l'addition d'hexane font précipiter le produit sous forme d'un solide jaune clair qui est filtré, lavé à l'hexane, séché sous vide (4,5 g, rendement = 76 %).
RMN ¹H (250 MHz, CDCl₃) δ, ppm : 8,48 (d,³*J*_{HH} = 5,5 Hz, 1H, H2'), 7,90 (d,⁴*J*_{HH} = 2,2 Hz, 1H, H8'), 7,70 (d,³*J*_{HH} = 8,9 Hz, 1H, H5'), 7,50 (s large, 1H, *H*N9'), 7,29 (dd,³*J*_{HH} = 8,9 Hz, ⁴*J*_{HH} = 2,2 Hz, 1H, H6'), 6,30 (d,³*J*_{HH} = 5,5 Hz, 1H, H3'), 3,39 (m, 2H, *H₂*C10'), 3,03 (tr, 2H, *H₂*C12'), 1,87 (m, 2H, *H₂*C11'), 1,58 (s large, *H₂*N13').
SM (DCI/NH₃⁺) *m*/*z* (%) : 235 (2), 236 (MH⁺, 100), 237 (14), 238 (34), 239 (5).

### Couplage de l'amine primaire avec la cétone par amination réductrice : obtention de la trioxaquine 6

La cétone **2** (199 mg, 0,55 mmol) et l'amine primaire 5 (165 mg, 0,70 mmol) sont mises en solution dans CH₂Cl₂ (10 mL). On ajoute le triacétoxyborohydrure de sodium (146 mg, 0,69 mmol). Le mélange est maintenu sous agitation à température ambiante pendant 15 heures. Le milieu réactionnel est ensuite lavé à l'eau distillée ; la phase organique est séchée et le solvant est évaporé à sec (rendement : 96 %).
RMN ¹H (250 MHz, CDCl₃) δ, ppm : 8,51 (2 x d, 1H, H2'), 8,04 (s large, 1H, *H*N9'), 7,90 (2 x d, 1H, H8'), 7,80 (2 x d, 1H, H5'), 7,65-7,25 (m, 11H, H6' et 10H phényle), 6,30 (m, 2H, H3' et H6), 5,14 (2 x s large, 1H, H5), 3,39 (q, 2H, *H₂*C10'), 3,29 (d, 1H, *H*C8), 2,95 (m, 3H, *H₂*C12' et *H*C8), 2,60 (m, 2H, cyclohexyle), 2,42 (m, 2H, *H*C12 et *H*N13'), 2,10-1,25 (m, 10H, 8H cyclohexyle et *H₂*C11').
SM (DCI/NH₃⁺) *mlz* (%) : 580 (5), 582 (MH⁺, 100), 583 (39), 584 (39), 585 (15).

### Obtention du dicitrate de trioxaquine 7

La trioxaquine **4** (81 mg, 0,14 mmol) est mise en solution dans l'acétone (4 mL). On ajoute de l'acide citrique (80 mg, 3,0 équiv.) en solution dans l'acétone (5 mL). Le dicitrate de trioxaquine précipite; il est centrifugé, lavé deux fois au diéthyl éther, séché sous vide.
RMN ¹H (250 MHz, DMSO-*d*₆) δ, ppm : 8,60 (2 x d, 1H, H2'), 8,42 (2 x d, 1H, H5'), 7,93 (2 x d, 1H, H8'), 7,65 (m, 5H, H6' et 4H phényle), 7,45 (m, 6H, phényle), 6,72 (2 x d, 1H, H3'), 6,61 (2 x q, 1H, H6), 5,43 (2 x s large, 1H, H5), 3,8-3,0 (m, 7H, *H₂*C10', *H₂*C12', *H₂*C8 et *H*C12), 2,76 (d, 4H, citrate), 2,65 (d, 4H, citrate), 2,20-1,40 (m, 10H, 8H cyclohexyle et *H₂*C11').

| | |
|---|---|
| SM (ES) *m*/*z* (%) : | en mode positif 582,3 (M⁺) |
| | en mode négatif 190,8 (citrate) |

| Microanalyse élémentaire: pour C₄₇H₅₂O₁₇N₃Cl, 1 H₂O | | | |
|---|---|---|---|
| % Théor. : | C 57,35 | H 5,53 | N 4,27 |
| % Expér. : | C 57,09 | H 5,20 | N 4,24 |

### Exemple 3 : trioxaquine 10

### Synthèse de la 7-chloro-4-[N-(4-aminobutyl)amino]-quinoléine 8

Un mélange de 4,7-dichloroquinoléine (5 g, 25 mmol) et de 1,4-diaminobutane (13 mL, 129 mmol), est chauffé au reflux de la diamine pendant 5 heures sous agitation magnétique. Après refroidissement, le solide obtenu est extrait par du dichlorométhane (3 x 100 mL) à reflux. La phase organique est lavée à l'eau distillée, séchée sur sulfate de sodium. La concentration de la phase dichlorométhane, puis l'addition d'hexane font précipiter le produit sous forme d'un solide jaune clair qui est filtré, lavé à l'hexane, séché sous vide (3,4 g, rendement = 54 %).
RMN ¹H (250 MHz, CDCl₃) δ, ppm : 8,50 (d,³*J*_{HH} = 5,5 Hz, 1H, H2'), 7,92 (d,⁴*J*_{HH} = 2,2 Hz, 1H, H8'), 7,72 (d,³*J*_{HH} = 8,9 Hz, 1H, H5'), 7,31 (dd,³*J*_{HH} = 8,9 Hz, ⁴*J*_{HH} = 2,2 Hz, 1H, H6'), 6,36 (d,³*J*_{HH} = 5,5 Hz, 1H, H3'), 6,04 (s large, 1H, *H*N9'), 3,29 (m, 2H, *H*₂C10'), 2,81 (tr, 2H, *H*₂C13'), 1,85 (m, 2H, *H*₂C11'), 1,64 (m, 2H, *H*₂C12'), 1,45 (s large, *H₂*N14'). SM (DCI/NH₃⁺) *m*/*z* (%) : 249 (2), 250 (MH⁺, 100), 251 (18), 252 (36), 253 (5).

### Couplage de l'amine primaire avec la cétone par amination réductrice : obtention de la trioxaquine 9

La cétone **2** (170 mg, 0,47 mmol) et l'amine primaire **8** (150 mg, 0,60 mmol) sont mises en solution dans CH₂Cl₂ (10 mL). On ajoute le triacétoxyborohydrure de sodium (125 mg, 0,59 mmol). Le mélange est maintenu sous agitation à température ambiante pendant 15 heures. Le milieu réactionnel est ensuite lavé à l'eau distillée ; la phase organique est séchée et le solvant est évaporé à sec (rendement : 69 %).
RMN ¹H (250 MHz, CDCl₃) δ, ppm : 8,50 (2 x d, 1H, H2'), 7,89 (2 x d, 1H, H8'), 7,78 (2 x d, 1H, H5'), 7,65-7,25 (m, 11H, H6' et 10H phényle), 6,35 (m, 2H, H3' et H6), 5,96 (s large, 1H, *H*N9'), 5,18 (2 x s large, 1H, H5), 3,30 (m, 3H. *H₂*C10' et *H*C8), 3,00 (2 x d, 1H, *H*C8), 2,74 (q, 2H, *H₂*C13'), 2,61 (m, 2H, cyclohexyle), 2,46 (m, 1H, *H*C12), 2,10-1,25 (m, 11H, 6H cyclohexyle, HN14', *H₂*C11' et *H₂*C12').
SM (DCI/NH₃⁺) *mlz* (%) : 596 (MH⁺).

### Obtention du dicitrate de trioxaquine 10

La trioxaquine **9** (51 mg, 0,09 mmol) est mise en solution dans l'acétone (1 mL). On ajoute de l'acide citrique (33 mg, 2,0 équiv.) en solution dans l'acétone (1 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,56 (2 x d, 1H, H2'), 8,45 (2 x d, 1H, H5'), 7,95 (m + 2 x d, 2H, HN9' et H8'), 7,68 (m, 5H, H6' et 4H phényle), 7,48 (m, 6H, phényle), 6,72 (2 x d, 1H, H3'), 6,60 (2 x q, 1H, H6), 5,41 (2 x s large, 1H, H5), 3,50 (m, 2H, *H₂*C10'), 3,55-3,10 (m, 5H, *H₂*C13', *H*₂C8 et *H*C12), 2,75 (d, 4H, citrate), 2,65 (d, 4H, citrate), 2,10-1,50 (12H, 8H cyclohexyle, *H₂*C11' et *H₂*C12').

| | |
|---|---|
| SM (ES) *m*/*z* (%) : | en mode positif 596,2 (M⁺) |
| | en mode négatif 190,8 (citrate) |

| Microanalyse élémentaire : pour C₄₈H₅₄O₁₇N₃Cl, 4 H₂O | | | |
|---|---|---|---|
| % Théor. : | C 54,78 | H 5,94 | N 3,99 |
| % Expér. : | C 54,88 | H 5,08 | N 4,06 |

### Exemple 4 : trioxaquines 13a et 13b

### Synthèse du trioxane fonctionnalisé par une cétone 11

Un mélange de 1,4-diphényl-1,3-cyclopentadiène (153 mg, 0,7 mmol) et de tétraphénylporphyrine (5 mg) dans du dichlorométhane (5 mL) est irradié en présence d'oxygène moléculaire (1,15 bar) pendant 1 heure, à 5 °C, avec une lampe blanche (200 W). Le peroxyde est obtenu avec un rendement quantitatif. Le peroxyde brut en solution dans le dichlorométhane est placé dans un bain à - 70 °C ; 4 équivalents molaires de cisbicyclo(3.3.0)octane-3,7-dione (410 mg, 3,0 mmol) et 0,4 équivalent de triméthylsilyl trifluorométhane sulfonate (50 µL, 0,3 mmol) sont ajoutés et le mélange réactionnel est maintenu sous agitation à - 70 °C pendant 2 heures. La réaction est arrêtée par addition de triéthylamine (100 µL). Après retour à la température ambiante, le milieu réactionnel est lavé à l'eau distillée, séché sur sulfate de magnésium et évaporé à sec. Une chromatographie sur colonne de silice (éluant hexane/acétate d'éthyle, 70/30, v/v) permet de séparer les deux trioxanes isomères **11a** et **11b** (rendement global : 42 %).

### Isomère 11a :

RMN ¹H (250 MHz, CDCl₃) δ, ppm : 7,60-7,30 (m, 10H, phényle), 6,29 (dd, 1H, H6), 5,27 (s large, 1H, H5), 3,21 et 3,02 (2 x d, ²*J*_{HH} = 17,0 Hz, 2 x 1H, *H₂*C8), 2,83 (m, 2H), 2,72 (m, 1H), 2,50 (m, 2H), 2,20 (m, 3H), 1,77 (m, 2H).
SM (DCI/NH₃⁺) *m*/*z* (%) : 406 (MNH₄⁺, 100), 407 (30), 408 (8).

### Isomère 11b :

RMN ¹H (250 MHz, CDCl₃) δ, ppm : 7,60-7,30 (m, 10H, phényle), 6,32 (dd, 1H, H6), 5,25 (s large, 1H, H5), 3,22 et 3,02 (2 x d, ²*J*_{HH} = 17 Hz, 2 x 1H, *H₂*C8), 2,90 (m, 2H), 2,50-2,15 (m, 7H), 1,79 (m, 1H).
SM (DCI/NH₃⁺) *m*/*z* (%) : 404 (3), 405 (3), 406 (MNH₄⁺, 100), 407 (31), 408 (6), 409 (1).

### Couplage de l'amine primaire avec la cétone par amination réductrice : obtention des trioxaquines 12a et 12b

La cétone **11a** (163 mg, 0,42 mmol) et l'amine primaire **1** (120 mg, 0,54 mmol) sont mises en solution dans CH₂Cl₂ (15 mL). On ajoute le triacétoxyborohydrure de sodium (114 mg, 0,54 mmol). Le mélange est maintenu sous agitation à température ambiante pendant plusieurs semaines. Le milieu réactionnel est ensuite lavé à l'eau distillée ; la phase organique est séchée et le solvant est évaporé à sec (rendement : 66 %).
RMN ¹H (250 MHz, CDCl₃) δ, ppm : 8,42 (d, 1H, H2'), 7,86 (d, 1H, H8'), 7,75 (d, 1H, H5'), 7,65-7,25 (m, 11H, H6' et 10H phényle), 6,30 (d, 1H, H3'), 6,23 (m, 1H, H6), 6,18 (s large, 1H, *H*N9'), 5,25 (s large, 1H, H5), 3,57 (s large, 1H, *H*N12'), 3,37 (m, 2H, *H*₂C10') *H*C8), 3,20 (m, 2H, *H*C8 et 1H bicyclopentyle), 3,00 (m, 3H, *H₂*C11' et *H*C8), 2,75 (m, 1H, bicyclopentyle), 2,45 (m, 2H, *H*C12 et 1H bicyclopentyle), 2,20 (m, 2H, bicyclopentyle), 1.74-1.10 (m, 5H, bicyclopentyle).
SM (DCI/NH₃⁺) *m*/*z* (%) : 594 (MH⁺).

La cétone **11b** (148 mg, 0,38 mmol) et l'amine primaire **1** (110 mg, 0,50 mmol) sont mises en solution dans CH₂Cl₂ (15 mL). On ajoute le triacétoxyborohydrure de sodium (154 mg, 0,73 mmol). Le mélange est maintenu sous agitation à température ambiante pendant une semaine. Le milieu réactionnel est ensuite lavé à l'eau distillée ; la phase organique est séchée et le solvant est évaporé à sec (rendement : 68 %).
RMN ¹H (250 MHz, CDCl₃) δ, ppm : 8,48 (d, 1H, H2'), 7,88 (d, 1H, H8'), 7,73 (d, 1H, H5'), 7,65-7,25 (m, 11H, H6' et 10H phényle), 6,27 (m, 2H, H3' et H6), 6,06 (s large, 1H, *H*N9'), 5,25 (s large, 1H, H5), 3,25 (m, 2H, *H₂*C10'), 3,21 (d, 1H, *H*C8), 3,01 (d, 1H, *H*C8), 2,94 (m, 3H, *H₂*C11' et 1H bicyclopentyle), 2,54 (m, 3H, *H*C12 et 2H bicyclopentyle), 2,10 (m, 4H, HN12' et 3H bicyclopentyle), 1,77 (m, 1H, bicyclopentyle), 1,25 (m, 3H, bicyclopentyle).
SM (DCI/NH₃⁺) *m*/*z* (%) : 594 (MH⁺ 100), 595 (44), 596 (44).

### Obtention des dicitrates de trioxaquine 13a et 13b

La trioxaquine **12a** (166 mg, 0,28 mmol) est mise en solution dans l'acétone (5 mL). On ajoute de l'acide citrique (160 mg, 3,0 équiv.) en solution dans l'acétone (5 mL). Le dicitrate de trioxaquine précipite ; il est centrifugé, lavé deux fois au diéthyl éther, séché sous vide. RMN ¹H (250 MHz, DMSO-*d*₆) δ, ppm : 8,63 (d, 1H, H2'), 8,40 (d, 1H, H5'), 8,00 (d, 1H, H8'), 7,70 (m, 5H, H6' et 4H phényle), 7,50 (m, 6H, phényle), 6,79 (d, 1H, H3'), 6,60 (q, 1H, H6), 5,53 (s large, 1H, H5), 3,75 (m, 3H, *H₂*C10' et *H*C8), 3,30 (m, 2H, *H*C8 et *H*C12), 3,12 (m, 2H, *H₂*C11'), 2,80 (d, 4H, citrate), 2,68 (d, 4H, citrate), 2,39 (m, 4H, bicyclopentyle), 2,10-1,50 (m, 6H, bicyclopentyle).

| | |
|---|---|
| SM (ES) *m*/*z* (%) : | en mode positif 594,3 (M⁺). |

| Microanalyse élémentaire : pour C₄₈H₅₂O₁₇N₃Cl, 1 H₂O | | | |
|---|---|---|---|
| % Théor. : | C 57,86 | H 5,46 | N 4,22 |
| % Expér. : | C 58,11 | H 5,02 | N 4,66 |

La trioxaquine **12b** (153 mg, 0,26 mmol) est mise en solution dans l'acétone (5 mL). On ajoute de l'acide citrique (150 mg, 3,0 équiv.) en solution dans l'acétone (5 mL). Le dicitrate de trioxaquine précipite ; il est centrifugé, lavé deux fois au diéthyl éther, séché sous vide.
RMN ¹H (250 MHz, DMSO-*d*₆) δ ppm : 8,60 (d, 1H, H2'), 8,34 (d, 1H, H5'), 7,95 (d, 1H, H8'), 7,73 (m, 5H, H6' et 4H phényle), 7,51 (m, 6H, phényle), 6,73 (d, 1H, H3'), 6,60 (q, 1H, H6), 5,55 (s large, 1H, H5), 3,69 (m, 3H, *H₂*C10' et *H*C8), 3,44 (m, 1H, *H*C12), 3,25 (m, 1H, *H*C8), 3,16 (m, 2H, *H*₂C11'), 2,77 (d, 4H, citrate), 2,65 (d, 4H, citrate), 2,55-2,05 (m, 6H, bicyclopentyle), 1,80-1,40 (m, 4H, bicyclopentyle).

| | |
|---|---|
| SM (ES) *m*/*z* (%) : | en mode positif 594,2 (M⁺) |
| | en mode négatif 190,9 (citrate) |

| Microanalyse élémentaire : pour C₄₈H₅₂O₁₇N₃Cl, 1 H₂O | | | |
|---|---|---|---|
| % Théor. : | C 57,86 | H 5,46 | N 4,22 |
| % Expér. : | C 58,19 | H 5,05 | N 4,17 |

### Exemple 5 : trioxaquines 15a et 15b

### Couplage de l'amine primaire avec la cétone par amination réductrice : obtention des trioxaquines 14a et 14b

La cétone **11a** (29 mg, 0,075 mmol) et l'amine primaire **8** (25 mg, 0,10 mmol) sont mises en solution dans CH₂Cl₂ (5 mL). On ajoute le triacétoxyborohydrure de sodium (21 mg, 0,10 mmol). Le mélange est maintenu sous agitation à température ambiante pendant 48 heures. Le milieu réactionnel est ensuite lavé à l'eau distillée ; la phase organique est séchée et le solvant est évaporé à sec (rendement : 64 %).
RMN ¹H (250 MHz, CDCl₃) δ, ppm : 8,45 (d, 1H, H2'), 7,92 (d, 1H, H8'), 7,75 (d, 1H, H5'), 7,65-7,25 (m, 11H, H6' et 10H phényle), 6,33 (m, 2H, H3' et *H*N9'), 6,27 (m, 1H, H6), 5,25 (s large, 1H, H5), 3,25 (m, 2H, *H*₂C10'), 3,19 (d, 1H, *H*C8), 3,00 (m, 2H, *H*C8 et 1H bicyclopentyle), 2,8-2,0 (m, 8H, 4H bicyclopentyle, *H*₂C13', *H*C12 et *H*N14'), 1,75-1,10 (m, 9H, 5H bicyclopentyle, *H*₂C11' et *H*₂C12').
SM (DCI/NH₃⁺) *m*/*z* (%) : 622 (MH⁺).

La cétone **11b** (26 mg, 0,070 mmol) et l'amine primaire **8** (21 mg, 0,084 mmol) sont mises en solution dans CH₂Cl₂ (5 mL). On ajoute le triacétoxyborohydrure de sodium (18 mg, 0,085 mmol). Le mélange est maintenu sous agitation à température ambiante pendant 48 heures. Le milieu réactionnel est ensuite lavé à l'eau distillée ; la phase organique est séchée et le solvant est évaporé à sec. Le mélange obtenu contient 70 % de la trioxaquine **12b** et est utilisé tel quel dans l'étape suivante.
RMN ¹H (250 MHz, CDCl₃) δ, ppm : 8,44 (d, 1H, H2'), 7,90 (d, 1H, H8'), 7,75 (d, 1H, H5'), 7,65-7,25 (m, 11H, H6' et 10H phényle), 6,30 (m, 3H, H3', H6 et *H*N9'), 5,24 (s large, 1H, H5), 3,25 (m, 2H, *H₂*C10'), 3,20 (d, 1H, *H*C8), 3,00 (m, 1H, *H*C8), 2,85 (m, 1H, bicyclopentyle), 2,65-2,0 (m, 9H, 5H bicyclopentyle, *H₂*C13', *H*C12 et *H*N14'), 1,8-1,6 (m, 5H, 1H bicyclopentyle, *H₂*C11' et *H₂*C12'), 1,24 (m, 3H, bicyclopentyle).

### Obtention des dicitrates de trioxaquine 15a et 15b

La trioxaquine **14a** (30 mg, 0,05 mmol) est mise en solution dans l'acétone (0,4 mL). On ajoute de l'acide citrique (22 mg, 2,4 équiv.) en solution dans l'acétone (0,4 mL). Le dicitrate de trioxaquine précipite ; il est centrifugé, lavé deux fois au diéthyl éther, et séché sous vide.
RMN ¹H (250 MHz, DMSO-*d*₆) δ, ppm : 8,59 (d, 1H, H2'), 8,48 (d, 1H, H5'), 8,17 (s large, 1H, *H*N9'), 7,97 (d, 1H, H8'), 7,70 (m, 5H, H6' et 4H phényle), 7,50 (m, 6H, phényle), 6,77 (d, 1H, H3'), 6,57 (q, 1H, H6), 5,50 (s large, 1H, H5), 3,51 (m, 3H, *H₂*C10' et *H*C8), 3,10 (m, 4H, *H₂*C13', *H*C8 et *H*C12), 2,78 (d, 4H, citrate), 2,67 (d, 4H, citrate), 2,37 (m, 4H, bicyclopentyle), 2,10-1.50 (m, 10H, 6H bicyclopentyle, *H₂*C11' et *H₂*C12').

| | |
|---|---|
| SM (ES) *m*/*z* (%) : | en mode positif 622,3 (M⁺) |
| | en mode négatif 191,2 (citrate) |

La trioxaquine **14b** (28 mg, mélange brut) est mise en solution dans l'acétone (1 mL). On ajoute de l'acide citrique (30 mg, 4,4 équiv.) en solution dans l'acétone (2 mL). Le dicitrate de trioxaquine précipite ; il est centrifugé, lavé deux fois au diéthyl éther, et séché sous vide.
RMN ¹H (250 MHz, DMSO-*d*₆) δ, ppm : 8,57 (d, 1H, H2'), 8,45 (d, 1H, H5'), 7,93 (d, 1H, H8'), 7,70 (m, 5H, H6' et 4H phényle), 7,51 (m, 6H, phényle), 6,70 (d, 1H, H3'), 6,61 (q, 1H, H6), 5,52 (s large, 1H, H5), 4,0-3,0 (m, 7H, *H₂*C10', *H₂*C13', *H₂*C8 et *H*C12), 2,75 (d, 4H, citrate), 2,65 (d, 4H, citrate), 2,55-2,05 (m, 6H, bicyclopentyle), 1,90-1,30 (m, 8H, 4H bicyclopentyle, *H₂*C11' et *H₂*C12').

| | |
|---|---|
| SM (ES) *m*/*z* (%) : | en mode positif 622,4 (M⁺) |
| | en mode négatif 191,2 (citrate) |

### Exemple 6 : trioxaquine 18

### Synthèse du trioxane fonctionnalisé par une cétone 16

Un mélange d' α-terpinène (420 mg, 3,0 mmol) et de tétraphénylporphyrine (5 mg) dans du dichlorométhane (5 mL) est irradié en présence d'oxygène moléculaire (1,15 bar) pendant 7 heures, à 5 °C, avec une lampe blanche (200 W). Le peroxyde est obtenu avec un rendement quantitatif. Le peroxyde brut en solution dans le dichlorométhane est placé dans un bain à -70 °C ; 6 équivalents molaires de 1,4-cyclohexadione (2,05 g, 18,3 mmol) et 0,4 équivalent de triméthylsilyl trifluorométhane sulfonate (200 µL, 1,1 mmol) sont ajoutés et le mélange réactionnel est maintenu sous agitation à - 70 °C pendant 2 heures. La réaction est arrêtée par addition de triéthylamine (400 µL). Après retour à la température ambiante, le milieu réactionnel est lavé à l'eau distillée, séché sur sulfate de sodium et évaporé à sec. Le trioxane fonctionnalisé 16 est purifié par chromatographie sur colonne de silice (éluant hexane/acétate d'éthyle, 85/15, v/v) (rendement : 38 %).
RMN ¹H (250 MHz, CDCl₃) δ, ppm : 5,40 (m, 1H, H6), 4,00 (m, 1H, H5), 2,67 (m, 1H), 2,41 (m, 4H), 2,22 (m, 4H), 2,00 (m, 3H), 1,50 (m, 1H), 0,99 (m, 9H).
SM (DCI/NH₃⁺) *m*/*z* (%) : 297 (26), 298 (MNH₄⁺, 100), 299 (48), 300 (8), 301 (1).

### Couplage de l'amine primaire avec la cétone par amination réductrice : obtention de la trioxaquine 17

La cétone **16** (113 mg, 0,40 mmol) et l'amine primaire **1** (115 mg, 0,52 mmol) sont mises en solution dans CH₂Cl₂ (10 mL). On ajoute le triacétoxyborohydrure de sodium (109 mg, 0,51 mmol). Le mélange est maintenu sous agitation à température ambiante pendant 20 heures. Le milieu réactionnel est ensuite lavé à l'eau distillée ; la phase organique est séchée et le solvant est évaporé à sec (rendement : 82 %).
RMN ¹H (250 MHz, CDCl₃) δ, ppm : 8,50 (2 x d, 1H, H2'), 7,92 (2 x d, 1H, H8'), 7,69 (2 x d, 1H, H5'), 7,35 (2 x dd, 1H, H6'), 6,36 (2 x d, 1H, H3'), 5,95 (s large, 1H, *H*N9'), 5,41 (m, 1H, H6), 4,00 (m, 1H, H5), 3,29 (m, 2H, *H₂*C10'), 3,02 (m, 2H, *H₂*C11'), 2,63 (m, 2H), 2,43 (m, 1H), 2,20-1,90 (m, 5H), 1,85 (m, 3H), 1,50 (m, 4H), 1,02 (m, 9H).
SM (DCI/NH₃⁺) *m*/*z* (%) : 484 (2), 485 (6), 486 (MH⁺, 100), 487 (36), 488 (42), 489 (12), 490 (2).

### Obtention du dicitrate de trioxaquine 18

La trioxaquine **17** (45 mg, 0,09 mmol) est mise en solution dans l'acétone (1 mL). On ajoute de l'acide citrique (53 mg, 3,0 équiv.) en solution dans l'acétone (1 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,62 (2 x d, 1H, H2'), 8,35 (2 x d, 1H, H5'), 7,97 (2 x d, 1H, H8'), 7,69 (2 x dd, 1H, H6'), 6,75 (2 x d, 1H, H3'), 5,45 (m, 1H, H6), 4,17 (m, 1H, H5), 3,75 (m, 2H, *H₂*C10'), 3,35 (m, 2H, *H₂*C11'), 3,05 (m, 1H), 2,76 (d, 4H, citrate), 2,65 (d, 4H, citrate), 2,29 (m, 3H), 2,07 (m, 4H), 1,72 (m, 3H), 1,57 (m, 3H), 1,10 (m, 9H).

| | |
|---|---|
| SM (ES) *m*/*z* (%) : | en mode positif 486,2 (M⁺) |

| Microanalyse élémentaire : pour C₃₉H₅₂O₁₇N₃Cl | | | |
|---|---|---|---|
| % Théor. : | C 53,84 | H 6,03 | N 4,83 |
| % Expér. : | C 54,25 | H 5,43 | N 5,04 |

### Exemple 7 : trioxaquine 20

### Couplage de l'amine primaire avec la cétone par amination réductrice : obtention de la trioxaquine 19

La cétone **16** (100 mg, 0,36 mmol) et l'amine primaire **8** (115 mg, 0,46 mmol) sont mises en solution dans CH₂Cl₂ (10 mL). On ajoute le triacétoxyborohydrure de sodium (101 mg, 0,48 mmol). Le mélange est maintenu sous agitation à température ambiante pendant 15 heures. Le milieu réactionnel est ensuite lavé à l'eau distillée ; la phase organique est séchée et le solvant est évaporé à sec (rendement: 62 %).
RMN ¹H (250 MHz, CDCl₃) δ, ppm : 8,47 (2 x d, 1H, H2'), 7,89 (2 x d, 1H. H8'), 7,74 (2 x d, 1H, H5'), 7,35 (2 x dd, 1H, H6'), 6,34 (2 x d, 1H, H3'), 5,9-5,7 (m, 1H, *H*N9'), 5,39 (m, 1H, H6), 4,00 (m, 1H, H5), 3,27 (m, 2H, *H₂*C10'), 2,70 (m, 2H, *H₂*C13'), 2,60-2,35 (m, 3H), 2,30-1,95 (m, 5H), 1,83 (m, 3H), 1,67 (m, 4H, *H₂*C11' et *H₂*C12'), 1,50 (m, 4H), 1,00 (m, 9H).
SM (DCI/NH₃⁺) *m*/*z* (%) : 514 (3), 515 (MH⁺, 100), 516 (28), 517 (36), 518 (11).

### Obtention du dicitrate de trioxaquine 20

La trioxaquine **19** (46 mg, 0,09 mmol) est mise en solution dans l'acétone (1 mL). On ajoute de l'acide citrique (44 mg, 2,6 équiv.) en solution dans l'acétone (1 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,56 (2 x d, 1H, H2'), 8,45 (2 x d, 1H, H5'), 7,95 (2 x d, 1H, H8'), 7,67 (2 x dd, 1H, H6'), 6,72 (2 x d, 1H, H3'), 5,45 (m, 1H, H6), 4,15 (m, 1H, H5), 3,50 (m, 2H, *H₂*C10'), 3,20 (m, 1H), 3,05 (m, 2H, *H₂*C13'), 2,76 (d, 4H, citrate), 2,65 (d, 4H, citrate), 2,29 (m, 3H), 2,07 (m, 4H), 1,80 (m, 7H), 1,55 (m, 3H), 1,10 (m, 9H).

| | |
|---|---|
| SM (ES) *m*/*z* (%) : | en mode positif 514,4 (M⁺) |

| Microanalyse élémentaire : pour C₄₁H₅₆O₁₇N₃Cl | | | |
|---|---|---|---|
| % Théor. : | C 54,83 | H 6,29 | N 4,68 |
| % Expér. : | C 54,32 | H 5,80 | N 4,52 |

### Exemple 8 : trioxaquines 23a et 23b

### Synthèse des trioxanes fonctionnalisés par une cétone 21a, 21b et 21c

Un mélange d'α-terpinène (320 mg, 2,76 mmol) et de tétraphénylporphyrine (5 mg) dans du dichlorométhane (10 mL) est irradié en présence d'oxygène moléculaire (1,15 bar) pendant 7 heures, à 5 °C, avec une lampe blanche (200 W). Le peroxyde est obtenu avec un rendement quantitatif Le peroxyde brut en solution dans le dichlorométhane est placé dans un bain à - 70 °C ; 4 équivalents molaires de cisbicyclo(3.3.0)octane-3,7-dione (1,62 g, 11,7 mmol) et 0,5 équivalent de triméthylsilyl trifluorométhane sulfonate (250 µL, 1,38 mmol) sont ajoutés et le mélange réactionnel est maintenu sous agitation à - 70 °C pendant 4 heures. La réaction est arrêtée par addition de triéthylamine (400 µL). Après retour à la température ambiante, le milieu réactionnel est lavé à l'eau distillée, séché sur sulfate de sodium et évaporé à sec. Une chromatographie sur colonne de silice (éluant hexane/acétate d'éthyle, 70/30, v/v) permet de séparer trois trioxanes isomères **21c, 21a** et **21b,** par ordre d'élution (rendement global : 35 %).

### Isomère 21a :

RMN ¹H (250 MHz, CDCl₃) δ, ppm : 5,37 (d large, 1H, H6), 3,85 (d large, 1H, H5), 3,10-2,60 (m, 3H), 2,48 (m, 2H), 2,16 (m, 6H), 1,90-1,40 (m, 4H), 0,99 (m, 9H).
SM (DCl/NH₃⁺) *m*/*z* (%) : 323 (4), 324 (MNH₄⁺, 100), 325 (21), 326 (5).

### Isomère 21b :

RMN ¹H (250 MHz, CDCl₃) δ, ppm : 5,38 (d large, 1H, H6), 3,88 (d large. 1H, H5), 2,82 (m, 2H), 2,62 (m, 1H), 2,5-2,0 (m, 10H), 1,72 (m, 1H), 1,49 (m, 1H), 0,99 (m, 9H).
SM (DCl/NH₃⁺) *m*/*z* (%) : 323 (14), 324 (MNH₄⁺, 100), 325 (23), 326 (5), 327 (1).

### Isomère 21c :

RMN ¹H (250 MHz, CDCl₃) δ, ppm : 5,23 (m, 1H, H6), 4,40 (m, 1H, H5), 3,10-2,6 (m, 3H), 2,49 (m, 2H), 2,17 (m, 6H), 1,9-1,5 (m, 4H), 1,37 (s, 3H), 1,00 (m, 6H).
SM (DCI/NH₃⁺) *m*/*z* (%) : 323 (26), 324 (MNH₄⁺, 100), 325 (19), 326 (5), 327 (1).

### Couplage de l'amine primaire avec la cétone par amination réductrice : obtention des trioxaquines 22a et 22b

La cétone **21a** (70 mg, 0,23 mmol) et l'amine primaire **1** (66 mg, 0,30 mmol) sont mises en solution dans CH₂Cl₂ (5 mL). On ajoute le triacétoxyborohydrure de sodium (61 mg, 0,29 mmol). Le mélange est maintenu sous agitation à température ambiante pendant une semaine. Le milieu réactionnel est ensuite lavé à l'eau distillée ; la phase organique est séchée et le solvant est évaporé à sec (rendement : 70 %).
RMN ¹H (250 MHz, CDCl₃) δ, ppm : 8,45 (d, 1H, H2'), 7,85 (d, 1H, H8'), 7,70 (d, 1H, H5'), 7,29 (dd, 1H, H6'), 6,31 (d, 1H, H3'), 6,09 (s large, 1H, *H*N9'), 5,36 (d large, 1H, H6), 3,87 (d large, 1H, H5), 3,31 (m, 2H, *H₂*C10'), 3,11 (m, 2H), 2,99 (m, 2H, *H₂*C11'), 2,75-2,35 (m, 3H), 2,20 (m, 6H), 1,90-1,40 (m, 4H), 1,21 (m, 2H), 0,98 (m, 9H).
SM (DCI/NH₃⁺) *m*/*z* (%) : 512 (MH⁺, 100), 513 (32), 514 (46), 515 (15), 516 (7).

La cétone **21b** (35 mg, 0,11 mmol) et l'amine primaire 1 (32 mg, 0,14 mmol) sont mises en solution dans CH₂Cl₂ (5 mL). On ajoute le triacétoxyborohydrure de sodium (30 mg, 0,14 mmol). Le mélange est maintenu sous agitation à température ambiante pendant plusieurs semaines. Le milieu réactionnel est ensuite lavé à l'eau distillée ; la phase organique est séchée et le solvant est évaporé à sec (rendement : 75 %).
RMN ¹H (250 MHz, CDCl₃) δ, ppm : 8,46 (d, 1H, H2'), 7,90 (d, 1H, H8'), 7,76 (d, 1H, H5'), 7,35 (dd, 1H, H6'), 6,34 (d, 1H, H3'), 6,03 (s large, 1H, *H*N9'), 5,42 (d large, 1H, H6), 3,89 (d large, 1H, H5), 3,26 (m, 2H, *H₂*C10'), 2,97 (m, 3H, *H₂*C11' et 1H), 2,7-1,9 (m, 11H), 1,70 (m, 2H), 1,50 (m, 2H), 1,24 (m, 2H), 0,98 (m, 9H).
SM(DC1/NH₃⁺) *m*/*z* (%):510(10),512(MH⁺, 100), 513(35), 514(51), 515(16), 516(7).

### Obtention des dicitrates de trioxaquine 23a et 23b

La trioxaquine **22a** (82 mg, 0,16 mmol) est mise en solution dans l'acétone (5 mL). On ajoute de l'acide citrique (90 mg, 2,9 équiv.) en solution dans l'acétone (5 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,62 (d, 1H, H2'), 8,40 (d, 1H, H5'), 7,97 (d, 1H, H8'), 7,69 (dd, 1H, H6'), 6,78 (d, 1H, H3'), 5,49 (d large, 1H, H6), 4,02 (d large, 1H, H5), 3,78 (m, 2H, *H*₂C10'), 3,65 (m, 1H), 3,32 (m, 2H, *H*₂C11'), 2,79 (d, 4H, citrate), 2,68 (d, 4H, citrate), 2,60-1,90 (m, 9H), 1,80 (m, 2H), 1,60 (m, 4H), 1,10 (m, 9H).

| | |
|---|---|
| SM (ES) *m*/*z* (%) : | en mode positif 512,3 (M⁺) |
| | en mode négatif 190,8 (citrate) |

| Microanalyse élémentaire : pour C₄₁H₅₄O₁₇N₃Cl | | | |
|---|---|---|---|
| % Théor. : | C 54,95 | H 6,08 | N 4,69 |
| % Expér. : | C 55,07 | H 6,15 | N 4,52 |

La trioxaquine **22b** (44 mg, 0,09 mmol) est mise en solution dans l'acétone (4 mL). On ajoute de l'acide citrique (50 mg, 3,0 équiv.) en solution dans l'acétone (4 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,62 (d, 1H, H2'), 8,36 (d, 1H, H5'), 7,96 (d, 1H, H8'), 7,70 (dd, 1H, H6'), 6,75 (d, 1H, H3'), 5,48 (d large, 1H, H6), 4,05 (d large, 1H, H5), 3,73 (m, 2H, *H₂*C10'), 3,45 (m, 1H), 3,29 (m, 2H, *H₂*C11'), 2,80 (d, 4H, citrate), 2,68 (d, 4H, citrate), 2,31 (m, 6H), 2,06 (m, 4H), 1,8-1,4 (m, 5H), 1,10 (m, 9H).

| | |
|---|---|
| SM (ES) *m*/*z* (%) : | en mode positif 512,5 (M⁺) |
| | en mode négatif 191,2 (citrate) |

| Microanalyse élémentaire : pour C₄₁H₅₄O₁₇N₃Cl, 2 H₂O | | | |
|---|---|---|---|
| % Théor. : | C 52,81 | H 6,27 | N 4,51 |
| % Expér. : | C 52,93 | H 5,49 | N 5,18 |

### Exemple 9 : trioxaquines 25a et 25b

### Couplage de l'amine primaire avec la cétone par amination réductrice : obtention des trioxaquines 24a et 24b

La cétone **21a** (70 mg, 0,23 mmol) et l'amine primaire **8** (71 mg, 0,28 mmol) sont mises en solution dans CH₂Cl₂ (5 mL). On ajoute le triacetoxyborohydrure de sodium (61 mg, 0,29 mmol). Le mélange est maintenu sous agitation à température ambiante pendant une semaine. Le milieu réactionnel est ensuite lavé à l'eau distillée ; la phase organique est séchée et le solvant est évaporé à sec (rendement : 51%).
RMN ¹H (250 MHz, CDCl₃) δ, ppm : 8,49 (d, 1H, H2'), 7,92 (m, 1H, H8'), 7,74 (m, 1H, H5'), 7,32 (m, 1H, H6'), 6,35 (m, 1H, H3'), 6,0-5,8 (m, 1H, *H*N9'), 5,39 (d large, 1H, H6), 3,88 (d large, 1H, H5), 3,26 (m, 2H, *H₂*C10'), 2,91 (m, 2H), 2,68 (m, 2H, *H₂*C11'), 2,48 (m, 3H), 2,20 (m, 6H), 1,90-1,40 (m, 8H), 1,22 (m, 2H), 0,97 (m, 9H).
SM (DCI/NH₃⁺) *m*/*z* (%): 538 (7), 539 (5), 540 (MH⁺, 100), 541 (37), 542 (70), 543 (21), 544(13).

La cétone **21b** (35 mg, 0,11 mmol) et l'amine primaire **8** (40 mg, 0,16 mmol) sont mises en solution dans CH₂Cl₂ (5 mL). On ajoute le triacétoxyborohydrure de sodium (33 mg, 0,16 mmol). Le mélange est maintenu sous agitation à température ambiante pendant plusieurs semaines. Le milieu réactionnel est ensuite lavé à l'eau distillée ; la phase organique est séchée et le solvant est évaporé à sec (rendement : 76 %).
RMN ¹H (250 MHz, CDCl₃) δ, ppm : 8,48 (d, 1H, H2'), 7,90 (d, 1H, H8'), 7,75 (d, 1H, H5'), 7,33 (dd, 1H, H6'), 6,33 (d, 1H, H3'), 6,02 (s large, 1H, *H*N9'), 5,41 (d large, 1H, H6), 3,90 (d large, 1H, H5), 3,25 (m, 2H, *H₂*C10'), 2,93 (m, 1H), 2,67 (m, 2H, *H₂*C11'), 2,45 (m, 3H), 2,20 (m, 6H), 2,00 (m, 2H), 1,8-1,6 (m, 6H), 1,48 (m, 1H), 1,25 (m, 2H), 1,00 (m, 9H).
SM (DCI/NH₃⁺) *m*/*z* (%) : 538 (10), 539 (9), 540 (MH⁺, 100), 541 (40), 542 (67), 543 (23), 544 (14).

### Obtention des dicitrates de trioxaquine 25a et 25b

La trioxaquine **24a** (63 mg, 0,12 mmol) est mise en solution dans l'acétone (5 mL). On ajoute de l'acide citrique (70 mg, 3,0 équiv.) en solution dans l'acétone (5 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,58 (d, 1H, H2'), 8,49 (d, 1H, H5'), 8,14 (s large, 1H, *H*N9'), 7,97 (d, 1H, H8'), 7,70 (dd, 1H, H6'), 6,77 (d, 1H, H3'), 5,48 (d large, 1H, H6), 4,03 (d large, 1H, H5), 3,50 (m, 2H, *H₂*C10'), 3,36 (m, 1H), 3,04 (m, 2H, *H₂*C13'), 2,75 (d, 4H, citrate), 2,65 (d, 4H, citrate), 2,60-1,95 (m, 9H), 1,90-1,45 (m, 10H), 1,10 (m, 9H).

| | |
|---|---|
| SM (ES) *m*/*z* (%) : | en mode positif 540,3 (M⁺) |
| | en mode négatif 191,2 (citrate) |

| Microanalyse élémentaire : pour C₄₃H₅₈O₁₇N₃Cl | | | |
|---|---|---|---|
| % Théor. : | C 55,88 | H 6,33 | N 4,55 |
| % Expér. : | C 55,35 | H 6,27 | N 4,37 |

La trioxaquine **24b** (47 mg, 0,09 mmol) est mise en solution dans l'acétone (4 mL). On ajoute de l'acide citrique (50 mg, 3,0 équiv.) en solution dans l'acétone (4 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,58 (d, 1H, H2'), 8,45 (d, 1H, H5'), 8,01 (s large, 1H *H*N9'), 7,95 (d, 1H, H8'), 7,69 (dd, 1H, H6'), 6,75 (d, 1H, H3'), 5,47 (d large, 1H H6), 4,05 (d large, 1H H5), 3,48 (m, 2H, *H₂*C10'), 3,35 (m, 1H), 3,03 (m, 2H *H₂*C13'), 2,76 (d, 4H, citrate), 2,65 (d, 4H, citrate), 2,30 (m, 6H), 2,06 (m, 4H), 1,9-1,4 (m, 9H), 1,09 (m, 9H).

| | |
|---|---|
| SM (ES) *m*/*z* (%) : | en mode positif 540,4 (M⁺) |
| | en mode négatif 191,0 (citrate) |

| Microanalyse élémentaire : pour C₄₃H₅₈O₁₇N₃Cl, 1 H₂O | | | |
|---|---|---|---|
| % Théor. : | C 54,80 | H 6,42 | N 4,46 |
| % Expér. : | C 54,93 | H 6,01 | N 4,44 |

### Exemple 10 : trioxaquines 28a et 28b

### Synthèse des trioxanes fonctionnalisés par une cétone 26a et 26b

Un mélange de 1,3 cyclohexadiène (400 mg, 5 mmol) et de tétraphénylporphyrine (5 mg) dans du dichlorométhane (10 mL) est irradié en présence d'oxygène moléculaire (1,15 bar) pendant 5 heures, à 5 °C, avec une lampe blanche (200 W). Le peroxyde est obtenu avec un rendement quantitatif. Le peroxyde brut en solution dans le dichlorométhane est placé dans un bain à - 70 °C ; 4 équivalents molaires de 1,4 cyclohexanedione (2,3 g, 20 mmol) et 0,5 équivalent de triméthylsilyl trifluorométhane sulfonate (500 µL, 2,8 mmol) sont ajoutés et le mélange réactionnel est maintenu sous agitation à - 70 °C pendant 4 heures. La réaction est arrêtée par addition de triéthylamine (1000 µL). Après retour à la température ambiante, le milieu réactionnel est lavé à l'eau distillée, séché sur sulfate de sodium et évaporé à sec. Une chromatographie sur colonne de silice (éluant hexane/acétate d'éthyle, 70/30, v/v) permet de séparer les deux trioxanes isomères **26a** et **26b** (rendement global : 2 %).

### Isomère 26a :

RMN ¹H (250 MHz, CDCl₃) δ, ppm : 5,70 (m, 1H, H6), 5,57 (m, 1H, H7), 4,50 (m, 1H, H5), 4,25 (ddd, 1H, H10), 2,68 (m, 1H), 2,45 (m, 5H), 2,32 (m, 2H), 2,03 (m, 2H), 1,90 (m, 1H), 1,55 (m, 1H).
SM (DCI/NH₃⁺) *m*/*z* (%) : 241 (2), 242 (MNH₄⁺, 100), 243 (16), 244 (5).

### Isomère 26b :

RMN ¹H (250 MHz, CDCl₃) δ, ppm : 6,00 (m, 1H), 5,73 (m, 1H), 4,50 (m, 1H), 4,20 (m, 1H), 2,60-1,80 (m, 12H).
SM (DCI/NH₃⁺) *m*/*z* (%) : 241 (2), 242 (MNH₄⁺, 100), 243 (17), 244 (5).

### Couplage da l'amine primaire avec la cétone par amination réductrice : obtention des trioxaquines 27a et 27b

La cétone **26a** (9 mg, 0,04 mmol) et l'amine primaire **1** (12 mg, 0,05 mmol) sont mises en solution dans CH₂Cl₂ (3 mL). On ajoute le triacétoxyborohydrure de sodium (21 mg, 0,10 mmol). Le mélange est maintenu sous agitation à température ambiante pendant 15 heures. Le milieu réactionnel est ensuite lavé à l'eau distillée ; la phase organique est séchée et le solvant est évaporé à sec (rendement : 35 %).
RMN ¹H (250 MHz, CDCl₃) δ, ppm : 8,49 (d, 1H, H2'), 7,93 (2 x d, 1H, H8'), 7,75 (2 x d, 1H, H5'), 7,37 (m, 1H, H6'), 6,35 (m, 2H, H3' et *H*N9'), 5,66 (d large, 1H, H6), 5,55 (d large, 1H, H7), 4,48 (d large, 1H, H5), 4,15 (d large, 1H, H10), 3,38 (m, 2H, *H₂*C10'), 3,10 (m, 2H, *H₂*C11'), 2,67 (m, 2H), 2,49 (m, 3H), 2,30 (m, 2H), 2,10-1,30 (m, 7H).
SM (DCI/NH₃⁺) *m*/*z* (%) : 430 (MH⁺, 100), 431 (30), 432 (47).

La cétone **26b** (7 mg, 0,03 mmol) et l'amine primaire **1** (15 mg, 0,07 mmol) sont mises en solution dans CH₂Cl₂ (5 mL). On ajoute le triacétoxyborohydrure de sodium (12 mg, 0,06 mmol). Le mélange est maintenu sous agitation à température ambiante pendant 34 heures. Le milieu réactionnel est ensuite lavé à l'eau distillée; la phase organique est séchée et le solvant est évaporé à sec (rendement: 45 %).
RMN ¹H (250 MHz. CDCl₃) δ, ppm : 8,50 (2 x d, 1H, H2'), 7,92 (2 x d, 1H, H8'), 7,70 (2 x d, 1H, H5'), 7,36 (2 x dd, 1H, H6'), 6,34 (2 x d, 1H, H3'), 6,06 (s large, 1H, *H*N9'), 5,99 (m, 1H, H6), 5,72 (m, 1H, H7),4,41 (m, 1H). 4,10 (m, 1H), 3,32 (m, 2H, *H₂*C10'), 3,05 (m, 2H, *H₂*C11'), 2,63 (m, 2H), 2,30 (m, 4H), 2,10 (m, 1H), 2,00-1,35 (m, 7H).
SM (DCI/NH₃⁺) *m*/*z* (%) : 428 (15), 429 (9), 430 (MH⁺, 100), 431 (30), 432 (49), 433 (14).

### Obtention du dicitrate de trioxaquine 28b

La trioxaquine **27b** (6 mg, 0,014 mmol) est mise en solution dans l'acétone (1 mL). On ajoute de l'acide citrique (10 mg, 3,7 équiv.) en solution dans l'acétone (1 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,65 (d, 1H, H2'), 8,37 (d, 1H, H5'), 7,98 (d, 1H, H8'), 7,69 (dd, 1H, H6'), 6,75 (d, 1H, H3'), 6,05 (m, 1H), 5,80 (m, 1H), 4,57 (m, 1H), 4,30 (m, 1H), 3,75 (m, 2H, *H₂*C10'), 3,34 (m, 3H, *H₂*C13' et 1H), 2,80 (d, 4H, citrate), 2,68 (d, 4H, citrate), 2,50-1,50 (m, 12H).

| | |
|---|---|
| SM (ES) *m*/*z* (%): | en mode positif 430,2 (M⁺) |
| | en mode négatif 191,2 (citrate) |

| Microanalyse élémentaire : pour C₃₅R₄₄O₁₇N₃Cl | | | |
|---|---|---|---|
| % Théor. : | C 51,65 | H 5,45 | N 5,17 |
| % Expér. : | C 51,69 | H 5,18 | N 4,66 |

### Exemple 11 : trioxaquine 32

### Synthèse de l'oxoaldéhyde 4-oxopentanal 29

A une suspension de pyridinium chlorochromate PCC (6,4 g, 30 mmol) dans du dichlorométhane (25 mL), on ajoute lentement le 3-acétylpropan-1-ol (2,0 g, 20 mmol). Le mélange réactionnel est laissé sous agitation à température ambiante pendant 2 heures puis est filtré sur un gel de silice à l'éther. Le résidu noir est lavé deux fois à l'éther et les phases éthérées filtrées sont rassemblées et évaporées. L'aldéhyde est obtenu sous forme d'un liquide foncé (pureté 80 %, rendement 84 %).
RMN ¹H (250 MHz, CDCl₃) δ, ppm : 9,75 (s, 1H), 2,70 (s large, 4H), 2,14 (s, 3H).

### Synthèse du trioxane fonctionnalisé par une cétone 30

Un mélange de 2,3-diméthylbut-2ène (270 mg, 3,2 mmol) et de tétraphénylporphyrine (5 mg) dans du dichlorométhane (5 mL) est irradié en présence d'oxygène moléculaire (1,15 bar) pendant 7 heures, à 5 °C, avec une lampe blanche (200 W). Le peroxyde est obtenu avec un rendement quantitatif Au peroxyde brut en solution dans le dichlorométhane, on ajoute 7 équivalents molaires de 4-oxopentanal **29** (2,2 g, 22 mmol) et quelques gouttes d'acide trifluoroacétique CF₃COOH. Le mélange est agité à température ambiante pendant 90 minutes puis 2 équivalents de N-iodosuccinimide (1,35 g, 6 mmol) sont ajoutés et le mélange est maintenu sous agitation à température ambiante et à l'abri de la lumière pendant 3 heures, au bout desquelles le milieu réactionnel est lavé au thiosulfate de sodium à 20%, puis à l'eau distillée. Après séchage sur sulfate de sodium et évaporation à sec, le trioxane fonctionnalisé 30 est purifié par chromatographie sur colonne d'alumine (éluant hexane/éther, 50/50, v/v) (rendement : 14 %).
RMN ¹H (250 MHz, CDCl₃) δ, ppm : 5,39 (t, 1H, H3), 3,31 (d, ²*J*_{HH} = 11 Hz, 1H, *H*C10), 3,12 (d, ²*J*_{HH} = 11 Hz, 1H, *H*C10), 2,55 (m, 2H, *H*₂C12), 2,15 (s, 3H, *H₃*C14), 1,83 (m, 2H, *H₂*C11), 1,48 (2 s, 6H, *H₃*C8 et *H₃*C7), 1,06 (s, 3H, *H₃*C9).
SM (DCI/NH₃⁺) *m*/*z* (%) : 359 (22), 360 (MNH₄⁺, 100), 361 (14), 362 (2).

### Couplage de l'amine primaire avec la cétone par amination réductrice : obtention de la trioxaquine 31

La cétone **30** (13 mg, 0,04 mmol) et l'amine primaire **1** (15 mg, 0,07 mmol) sont mises en solution dans CH₂Cl₂ (4 mL). On ajoute le triacétoxyborohydrure de sodium (11 mg, 0,05 mmol). Le mélange est maintenu sous agitation à température ambiante pendant 7 jours. Le milieu réactionnel est ensuite lavé à l'eau distillée ; la phase organique est séchée et le solvant est évaporé à sec (rendement : 59 %).
RMN ¹H (250 MHz, CDCl₃) δ, ppm : 8,50 (d, 1H, H2'), 7;95 (d, 1H, H8'), 7,72 (d, 1H, H5'), 7,39 (2 x dd, 1H, H6'), 6,37 (2 x d, 1H, H3'), 6,00 (s large, 1H, *H*N9'), 5,37 (t, 1H, H3), 3,30 (m, 3H, *H₂*C10' et *H*C10), 3,11 (d, ²*J*_{HH} = 11 Hz, 1H, *H*C10), 3,00 (m, 2H, *H₂*C11'), 2,74 (m, 1H, *H*Cl3), 1,8 (s large, 1H, *H*N12'), 1,65-1,55 (m, 4H, *H₂*C11 et *H₂*C12), 1,50 (2 s, 6H, *H₃*C8 et *H₃*C7), 1,10 (d, 3H, *H₃*C14*),* 1,05 (s, 3H, *H₃*C9).
SM (DCI/NH₃⁺) *m*/*z* (%) : 547 (6), 548 (MH⁺, 100), 549 (29), 550 (37), 551 (9).

### Obtention du dicitrate de trioxaquine 32

La trioxaquine **31** (40 mg, 0,073 mmol) est mise en solution dans l'acétone (1 mL). On ajoute de l'acide citrique (52 mg, 3,7 équiv.) en solution dans l'acétone (1 mL). Le dicitrate de trioxaquine précipite : il est centrifugé, lavé deux fois au diéthyl éther, séché sous vide.
RMN ¹H (250 MHz, DMSO-*d*₆) δ, ppm : 8,63 (d, 1H, H2'), 8,37 (d, 1H, H5'), 7,99 (d, 1H, H8'), 7,71 (dd, 1H, H6'), 6,77 (d, 1H, H3'), 5,37 (t, 1H, H3), 3,70 (m, 4H, *H*₂C10', *H*C10 et *H*C13), 3,34 (m, 3H, *H*₂C11' et *H*C10), 2,78 (d, 4H, citrate), 2,67 (d, 4H, citrate), 2,0-1,6 (m, 4H), 1,54-1,35 (m, 6H), 1,22 (m, 6H).

| | |
|---|---|
| SM (ES) *m*/*z* (%) : | en mode positif 548,2 (M⁺) |
| | en mode négatif 191,2 (citrate) |

| Microanalyse élémentaire : pour C₃₄H₄₇O₁₇N₃ClI | | | |
|---|---|---|---|
| % Théor. : | C 43,81 | H 5,08 | N 4,51 |
| % Expér. : | C 44,08 | H 4,69 | N 4,72 |

### Exemple 12 : trioxaquine 34

### Couplage de l'amine primaire avec la cétone par amination réductrice : obtention de la trioxaquine 33

La cétone **30** (23 mg, 0,07 mmol) et l'amine primaire **8** (27 mg, 0,11 mmol) sont mises en solution dans CH₂Cl₂ (5 mL). On ajoute le triacétoxyborohydrure de sodium (27 mg, 0,13 mmol). Le mélange est maintenu sous agitation à température ambiante pendant 10 jours. Le milieu réactionnel est ensuite lavé à l'eau distillée ; la phase organique est séchée et le solvant est évaporé à sec (rendement : 10 %).
RMN ¹H (250 MHz, CDCl₃) δ, ppm : 8,50 (d, 1H, H2'), 7,90 (d, 1H, H8'), 7,75 (d, 1H, H5'), 7,34 (2 x dd, 1H, H6'), 6,38 (2 x d, 1H, H3'), 6,00-5,70 (s large, 1H, *H*N9'), 5,35 (t, 1H, H3), 3,30 (m, 3H, *H₂*C10' et *H*C10), 3,11 (d, ²*J*_{HH} = 11 Hz, 1H, *H*C10), 2,71 (m, 2H, *H₂*C13'), 2,51 (m, 1H, *H*C13), 1,78 (m, 4H), 1,65 (m, 4H), 1,48 (2 s, 6H, *H₃*C8 et *H₃*C7), 1,25 (m, 3H), 1,09 (s, 3H, *H₃*C9).
SM (DCI/NH₃⁺) *m*/*z* (%) : 576 (MH⁺).

### Obtention du dicitrate de trioxaquine 34

La trioxaquine **33** (20 mg, 0,035 mmol) est mise en solution dans l'acétone (1 mL). On ajoute de l'acide citrique (24 mg, 4,5 équiv.) en solution dans l'acétone (1 mL). Le dicitrate de trioxaquine précipite ; il est centrifugé, lavé deux fois au diéthyl éther, séché sous vide.
RMN ¹H (250 MHz, DMSO-*d*₆) δ, ppm : 8,62 (d, 1H, H2'), 8,46 (d, 1H, H5'), 7,96 (m, 2H, H8' et *H*N9'), 7,70 (m, 1H, H6'), 6,73 (d, 1H, H3'), 5,50 (t, 1H, H3), 4,0-3,0 (m, 7H, *H₂*C10', *H₂*C13', *H₂*C10 et *H*C13), 2,77 (d, 4H, citrate), 2,66 (d, 4H, citrate), 1,9-1,6 (m, 8H), 1,53 (m, 6H), 1,4-1,1 (m, 6H).
SM (ES) *m*/*z* (%) : en mode positif 576,2 (M⁺).

### Exemple 13 : Étude de l'activité antipaludique de trioxaquines sur P. falciparum.

On rapporte ci-après les résultats obtenus *in vitro* sur *P. falciparum* cultivé dans des hématies humaines.

### Partie expérimentale

Les souches de *P. falciparum* sont cultivées en continu selon la méthode de Trager et Jensen(5), avec les modifications suivantes (6) : les parasites sont maintenus dans des globules rouges humains (O±), dilués à 1% d'hématocrite dans un milieu RPMI 1640 supplémenté avec 25 mM d'Hépès + 30 mM NaHCO₃ et complémenté avec 5% de sérum humain AB+. Les populations de parasites sont synchronisées sur une période de 4 h par flottation avec une solution de gélatine, puis par lyse avec du D-sorbitol à 5% (7,8). La souche nigériane est considérée comme chloroquino-sensible, et les souches FcM29-Cameroon et FcBl-Coluumbia sont chloroquino-résistantes (CI₅₀ pour la chloroquine > 100 nM) (9,10). Les tests d'activité anitpaludique sont effectués par la microméthode radioactive de Desjardins (11). Les essais sont effectués en triple exemplaire, en microplaques de 96 puits, les lectures étant à 1% d'hématocrite et 0,5-1% de parasitémie. Pour chaque essai, les parasites sont incubés avec des concentrations décroissantes de drogue pendant soit 32 h, soit 72 h (à titre de référence, 4 puits contiennent de la chloroquine disphosphate). La première dilution de la drogue est effectuée à 10 mg/mL dans du diméthylsulfoxyde, et les dilutions suivantes sont effectuées avec du RPMI 1640. La croissance des parasites est mesurée par l'incorporation d'hypoxanthine tritiée comparée à l'incorporation en l'absence de drogue (prise comme 100%) (12), et les valeurs de CI₅₀ sont déterminées graphiquement en traçant le pourcentage d'inhibition en fonction de la concentration en drogue. Les IC₅₀ mesurées à 32 h, temps proche de la fin du stade trophozoïte, permettent d'évaluer l'influence du composé sur la maturation du parasite , les IC₅₀ mesurées après 72 h, durée d'1,5 cycle de vie du parasite dans les globules rouges, indiquent un effet possible sur la réinvasion des érythrocytes.

### Structures des trioxaquines testées :

### Résultats

Les trioxaquines 9, 3 et 6, ainsi que la trioxaquine citrate 4 ont été testées indépendamment sur les souches nigériane, FcB1 et FcM29 ; les résultats obtenus ont été comparés à ceux de la chloroquine disphosphate, et à ceux des deux fragments de trioxaquines : la quinoléine-amine 1 (n = 2) et le trioxane-cétone 2.

### Les résultats sont résumé dans le tableau suivant :

**Tableau.** Valeurs de CI₅₀ mesurées pour 9, 3, 6, 4 testés indépendamment sur trois souches de *Plasmodium falciparum.*

| | | FcB1-Colombia (CQR)^{a} | | FcM29-Cameroon (CQR+)^{a} | | Nigérian (CQS)^{a} | |
|---|---|---|---|---|---|---|---|
| | | 32h | 72h | 32h | 72h | 32h | 72h |
| 6 | ng/mL | 40 | 35 | 25 | 10 | 50 | 50 |
| (n=3) | nM | 69 | 60 | 43 | 17 | 86 | 86 |
| 9 | ng/mL | 16 | 10 | 22 | 20 | 20 | 20 |
| (n=4) | nM | 27 | 17 | 37 | 34 | 34 | 34 |
| 3 | ng/mL | 15 | 5 | 18 | 10 | 1 | 1 |
| (n=2) | nM | 23 | 9 | 32 | 18 | 1,8 | 1,8 |
| 4 | ng/mL | 35 | 20 | n.d.b | n.d.b | 33 | 7 |
| (n = 2), citrate | nM | 37 | 21 | | | 35 | 8 |
| Quinoléine-amine 1 | ng/mL | 35 | 20 | n.d.^{b} | n.d.^{b} | 33 | 7 |
| Trioxane-cétone 2 | nM | 113 | 36 | n.d.^{b} | n.d.^{b} | 149 | 45 |
| Chloroquine | ng/mL | 75 | 60 | 100 | 80 | 45 | 10 |
| disphophate^{c} | nM | 145 | 116 | 194 | 155 | 87 | 19 |

| | | | | | | | |
|---|---|---|---|---|---|---|---|
| ^{a} CQR = souche résistante à la chloroquine. CQR + = souche très résistante à la chloroquine, CQS = souche sensible à la chloroquine. ^{b} Non déterminé. ^{c}Donné pour comparaison. | | | | | | | |

Les valeurs de CI₅₀ obtenues pour les composés 3, 4, 6 et 9 se situent entre 5 et 50 ng/mL, ce qui correspond à des concentration de 8 à 86 nM.

Tous les échantillons de trioxaquines sont plus actifs que la chloroquine, que ce soit sur les souches sensibles ou résistantes (à l'exception de 6 sur la souche sensible nigériane). L'efficacité élevée de 9, 3 et 4 sur les souches sensibles et résistantes, largement supérieure à l'activité non seulement de la chloroquine, mais également de chacun des deux fragments quinoléine-amine 1 (n =2) et trioxane-cétone 2, indique un effet synergique important entre les fragments trioxane et chloroquinoléine des ces composés.

La forme citrate du composé 4 augmente notablement sa solubilité en milieu aqueux tout en lui conservant une activité élevée. La protonation par d'autres acides que l'acide citrique conduit à des sels présentant les mêmes avantages (chlorhydrate, sulfate, tartrate).

Les valeurs de CI₅₀ faibles obtenues avec la trioxaquine 3 sur les souches FcB1, FcM29 et sur la souche nigériane (9 nM, 18 nM et 1.8 nM respectivement), ainsi qu'avec 9 et le citrate 4, indiquent que l'efficacité de ces composés est conservée pour un large spectre de parasites.

### Exemple 14 : Préparation de compositions pharmaceutiques à base de molécules selon l'invention.

- sous forme de comprimés sécables
   - principe actif: 100 mg
   - excipients : amidon, silice hydratée, sucre pulvérisé amylacé, gélatine, stéarate de magnésium.
- sous forme de comprimés pelliculés
   - principe actif : 300 mg
   - excipients : Noyau : amidon de blé, sucre pulvérisé amylacé (saccharose pulvérulent additionné d'amidon) silice hydratée, gélatine, stéarate de magnésium. Enrobage : méthylhydroxy propylcellulose, poloxyéthylèneglycol 20 000.
- sous forme de sirops
   - principe actif : 25 mg/mL
   - excipients : acide citrique, saccharose, extrait de café, caramel, eau purifiée.
- sous forme de solutions injectables
   - principe actif : 100 mg pour une ampoule de 1 ml, 200 mg pour une ampoule de 2 ml, 400 mg pour une ampoule de 4 ml,
   - excipients : chlorure de sodium et eau pour préparations injectables.
- sous forme de solutions injectables à 25%
   - principe actif : 500 mg
   - excipients : acide lactique (solubilisant), acide formique, eau pour préparations injectables.

Conservateur : sulfite de sodium anhydre correspondant en anhydride sulfureux à 0,61 mg/amp
- sous forme de suspensions buvables à 100 mg/ml :
   - principe actif : 20 mg/ml
   - excipients : cellulose microcristalline et carboxyméthylcellulose sodique, propylèneglycol, sorbitol, acide citrique anhydre, citrate de sodium, benzoate de sodium, arôme banane-vanille, diméthylpolysiloxane émulsion, eau purifiée.

### Exemple 15 :

### Activité de la trioxaquine-citrate 4 sur isolats humains

La trioxaquine-citrate 4, dénommée DU-1102, a été testée sur isolats humains de *Plasmodium falciparum* dont certains étaient chloroquino- et/ou pyriméthamine-résistants. Les valeurs obtenues de concentration inhibitrices 50, Cl₅₀, sont de 11 à 68 ng/mL, correspondant à une moyenne géométrique de 41 ng/mL, soit 43 nM.

L'activité de DU-1102 sur ces isolats est indépendante de leur sensibilité ou de leur résistance aux autres antipaludiques testés.

II n'y a pas de corrélation entre DU-1102 d'une part et la chloroquine ou la pyriméthamine d'autre part, ce qui indique l'absence de probabilité de résistance croisée entre DU-1102 et ces antipaludiques déjà utilisés.

Ces résultats indiquent la bonne efficacité des trioxaquines sur des souches sauvages de *P. falciparum.*

### Activité de la trioxaquine-citrate DU-1302, représentées ci-dessous :

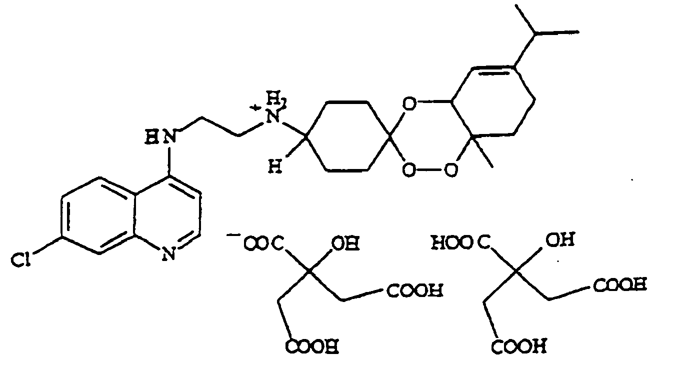

**2.1.** *In vitro,* ce composé présente une activité, CL₅₀ = 6 nM, sur *P. falciparum* en culture.
**2.2.** La trioxaquine-citrate DU-1302 est active in vivo chez la souris infectée par *P. vinckei* (test dit "de 4 jours" : traitement 4 jours consécutifs débutant 24 heures après inoculation du parasite),
   - DE₅₀ = 5 mg/kg/j, soit 6 µmol/kg/j, par voie intra-péritonéale, sans recrudescence observable durant 2 mois,
   - DE₅₀ = 18 mg/kg/j, soit 20 µmol/kg/j, par voie orale.
**2.3.** La trioxaquine-citrate DU-1302 ne présente pas de toxicité apparente après administration à des souris saines, à la dose de 100 mg/kg/j par voie orale, pendant trois jours consécutifs.

### 3. Absence de mutagénicité des trioxaquines-citrates DU-1102 et DU-1302

Les deux composés ci-dessus n'induisent pas la réparation de l'ADN (pas de réponse SOS dans *E.coli* GE864 à des concentrations de 5, 10, 15 et 20 µM : le témoin utilisé est la mitomycine C à 3 µM). Ils sont donc non-mutagènes pour *E.coli* à ces concentrations.

### Formules des composés dont la synthèse est décrite dans les exemples

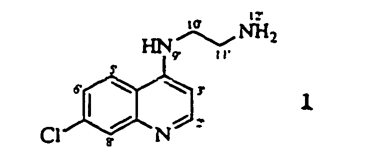

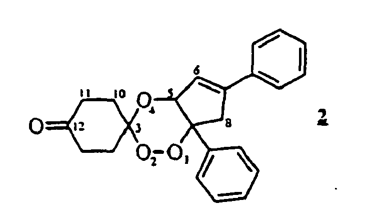

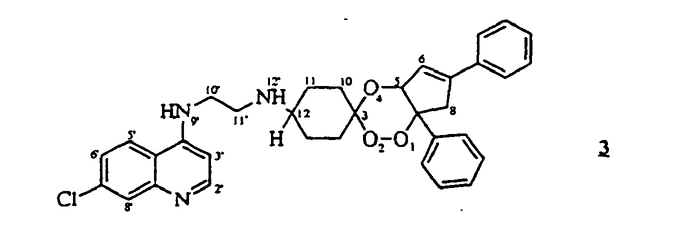

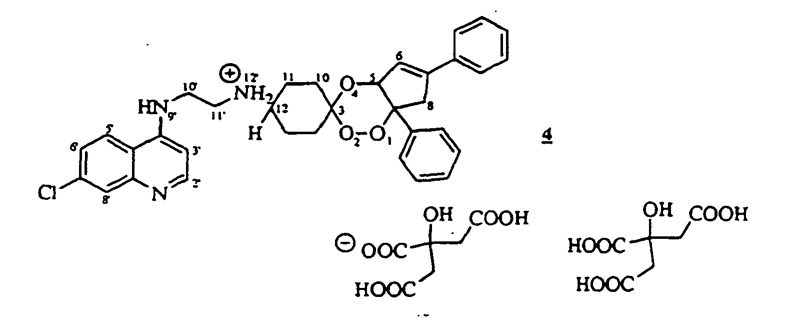

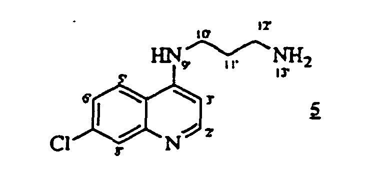

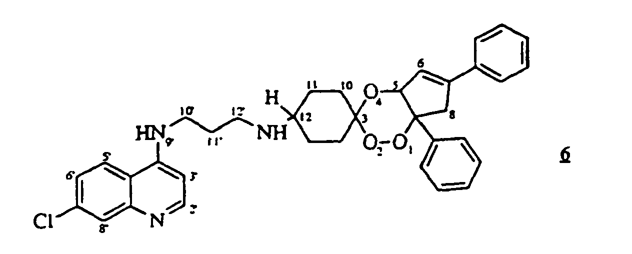

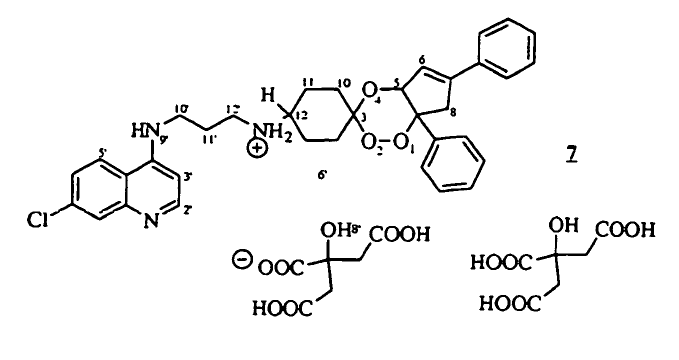

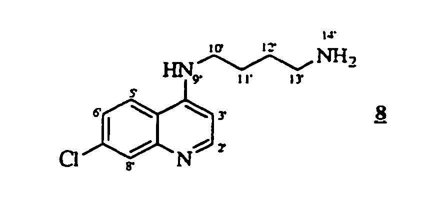

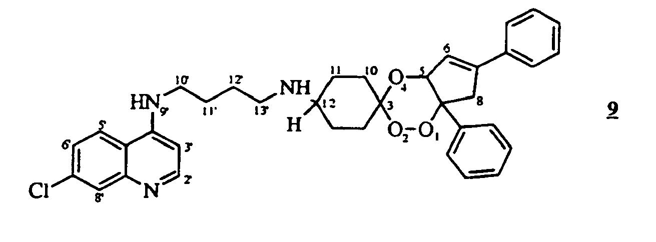

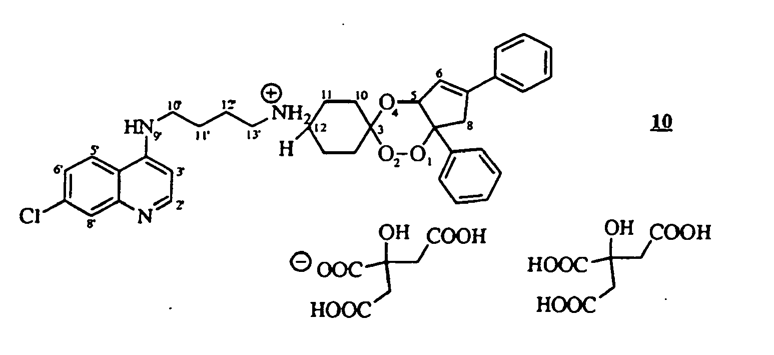

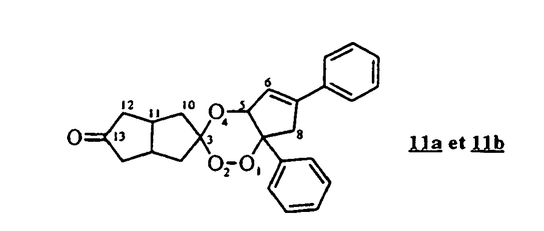

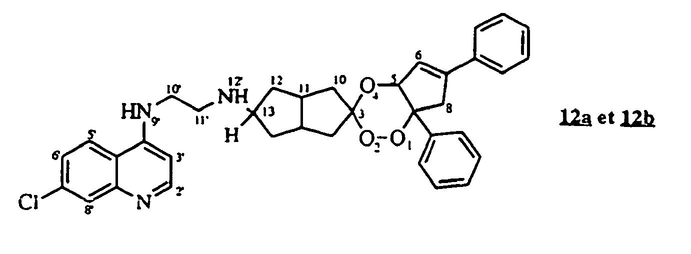

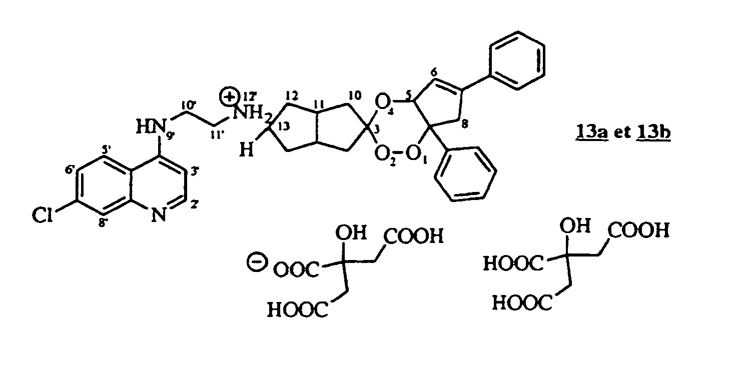

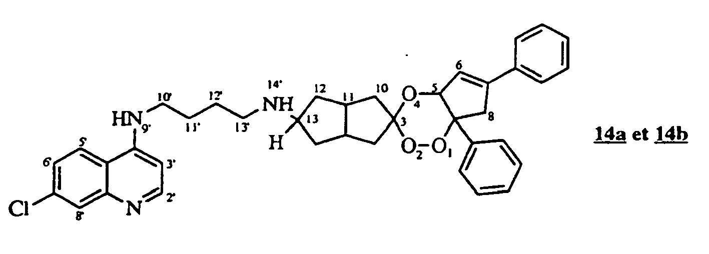

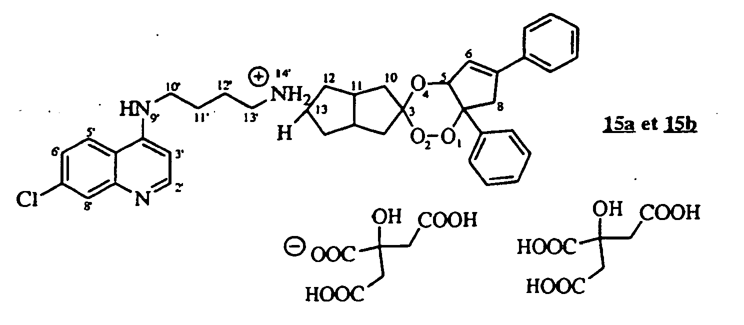

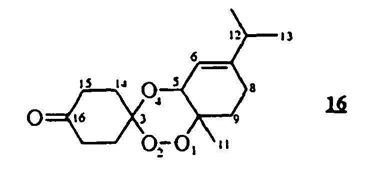

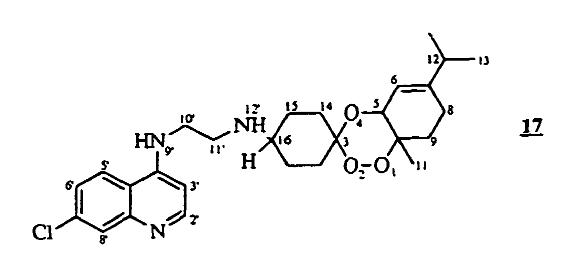

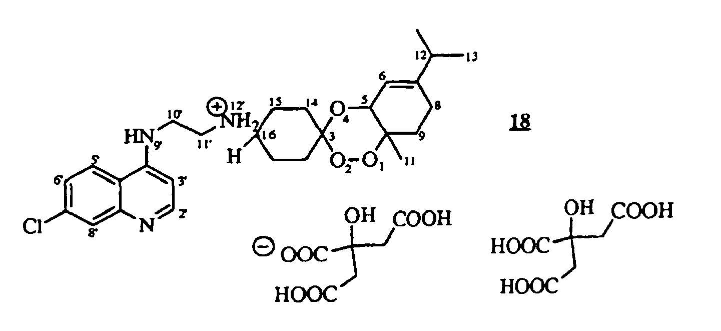

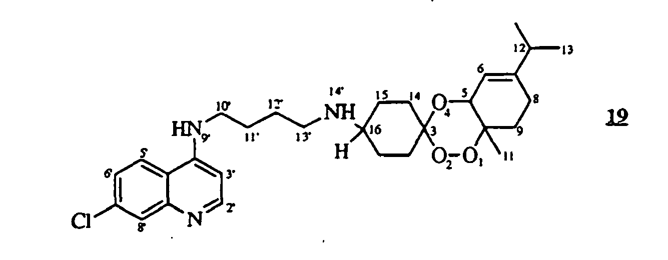

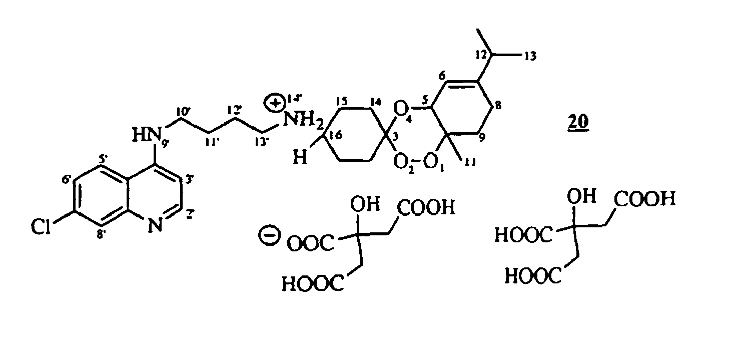

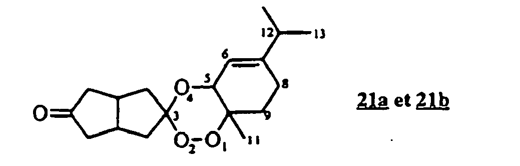

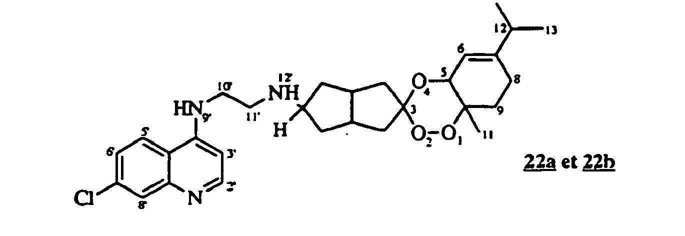

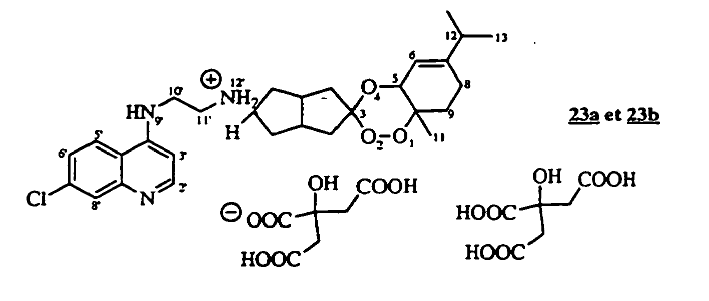

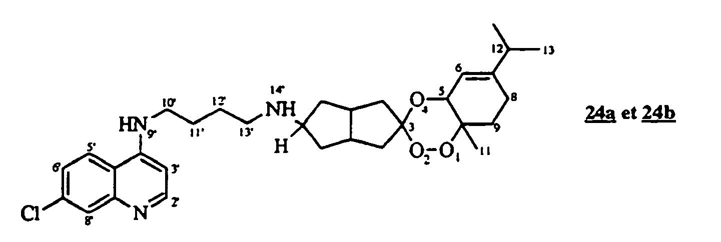

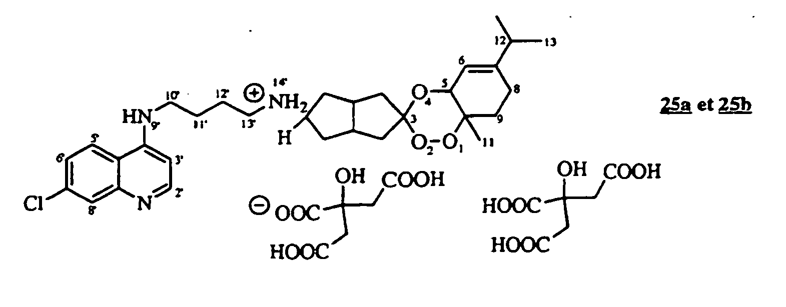

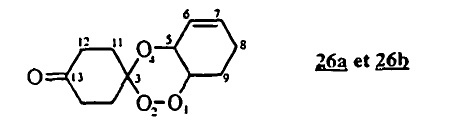

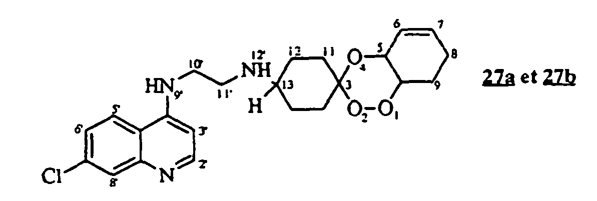

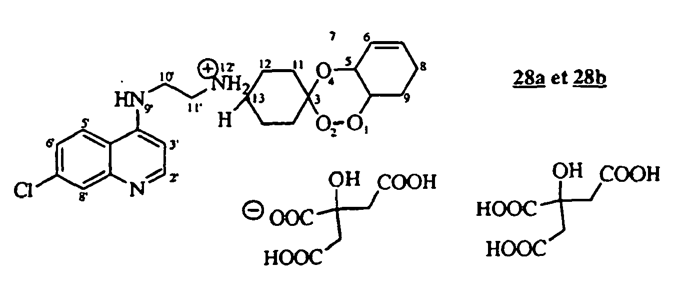

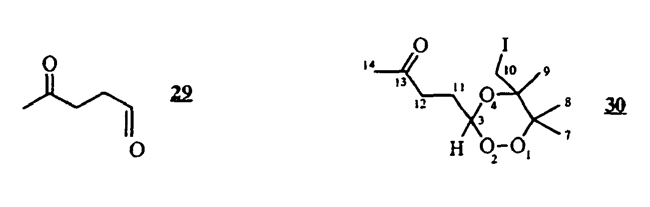

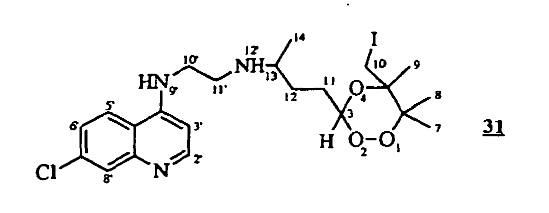

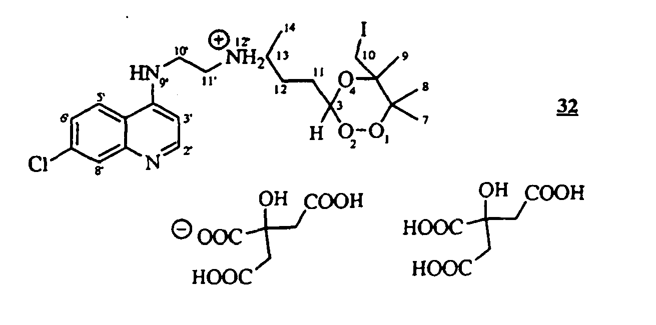

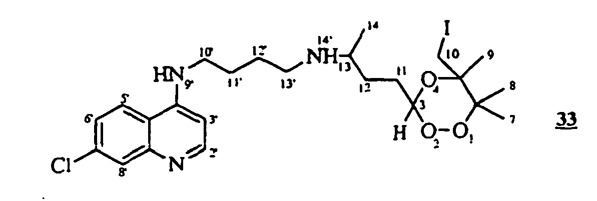

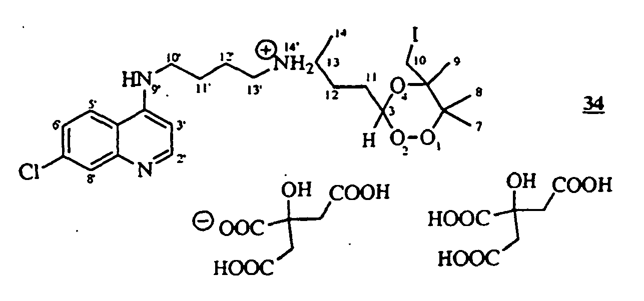

## Revendications

1. Molécules duales **caractérisées en ce qu'**il s'agit de produits de couplage répondant à la formule (I) 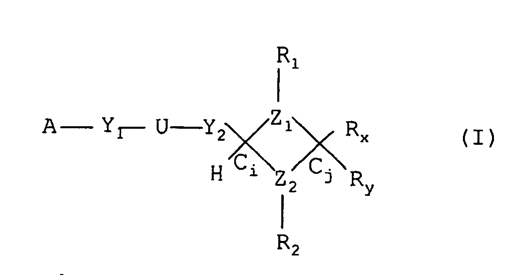 dans laquelle
- A représente un résidu de molécule à activité antipaludique du groupe comprenant
. un hétérocycle azoté, choisi parmi une aminoquinoléine de formule (II) ou une 1,5-naphtyridine de formule (III) suivantes 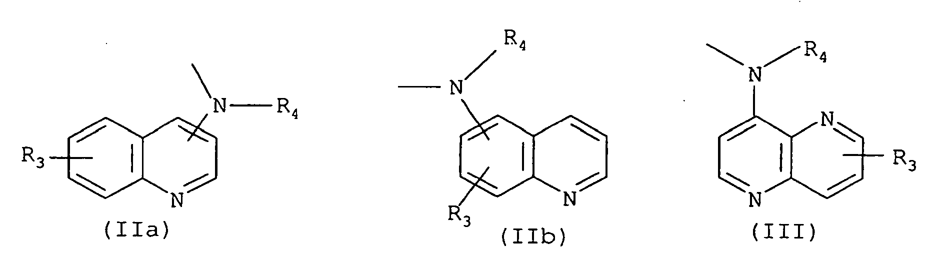
dans lesquelles
- R₃ représente, un ou plusieurs substituants identiques ou différents, occupant des positions distinctes, l'un au moins représentant un atome d'halogène, un groupe -OH, un groupe -CF₃, un radical aryle, un radical alkyle ou alkoxy en C1 à C5, un groupe -NO₂, le ou les autres substituants présentant l'une de ces significations ou un atome d'hydrogène,
- R₄ représente un radical alkyle en C1 à C5, linéaire, ramifié ou cyclique, ou un atome d'hydrogène,
un radical de formule **(IV)** R₅-CHOH dans laquelle R₅ représente un radical aryle ou un hétérocycle azoté,
. un résidu phénol-2 (aminométhyl) de formule (V) 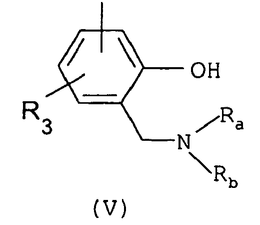
dans laquelle R₃ est tel que défini ci-dessus et Rₐ et R_{b}, identiques ou différents, représentent un atome d'hydrogène ou un radical alkyle en C1 à C5
. un résidu biguanide choisi parmi les dérivés de proguanil de formule (VI) 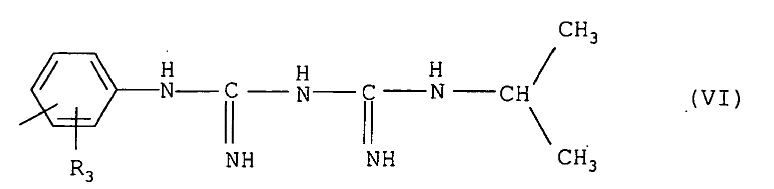
ou de cycloguanil de formule (VII) 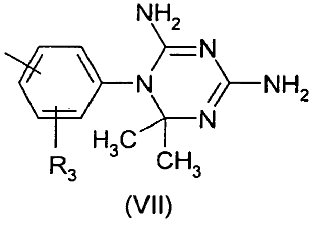 dans laquelle R₃ est tel que défini ci-dessus,
- A représente un résidu de pyrimidine et plus particulièrement de pyriméthamine de formule (VIII) 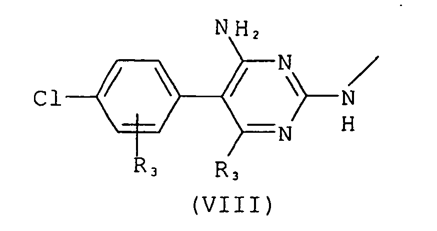
ou de formule (IX) 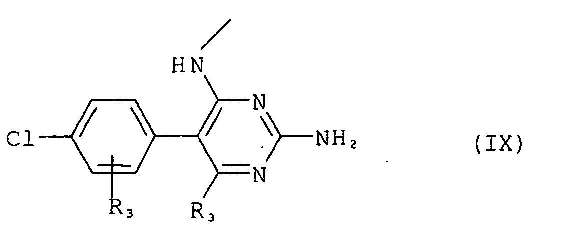 dans laquelle R₃ est tel que défini ci-dessus.
. un résidu acridine de formule (X) 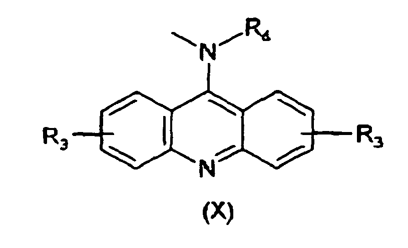
dans laquelle R₃ et R₄ sont tels que définis ci-dessus,
- Y₁ et Y₂, identiques ou différents, représentent une chaîne alkylène en C1 à C5. linéaire ou ramifiée, contenant le cas échéant un ou plusieurs radicaux amine, amide, sulfonamide, carboxyle, hydroxyle, éther ou thioéther, cette chaîne alkylène en C1 à C5 étant le cas échéant substituée par un radical alkyle en C1 à C5, l'un de Y₁ ou Y₂ pouvant être absent.
- U est une fonction amine, amide, sulfonamide, carboxyle, éther ou thioéther, cette fonction assurant le lien entre Y₁ et Y₂,
- Z₁ et Z₂, identiques ou différents, représentent un radical arylène ou alkylène linéaire, ramifié ou cyclique, saturé ou insaturé, l'un de Z₁ ou Z₂ pouvant être absent, ou l'ensemble Z₁ + Z₂ représente une structure polycyclique incluant les carbones de jonction. Ci et Cj,
- R₁ et R₂ identiques ou différents représentent un atome d'hydrogène ou un groupe fonctionnel capable d'augmenter l'hydrosolubilité de la molécule duale, avantageusement choisi parmi -COOH. -OH. -N(Rₐ, R_{b}) où Rₐ et R_{b} sont tels que définis ci-dessus.
- Rₓ et R_{y} forment un peroxyde cyclique de 4 à 8 chaînons, pouvant comporter 1 ou 2 atomes d'oxygène supplémentaires dans la structure cyclique, Cj étant l'un des sommets de ce peroxyde cyclique, ou
- Rₓ ou R_{y} est un peroxyde cyclique de 4 à 8 chaînons, pouvant comporter 1 ou 2 atomes d'oxygène supplémentaires dans Ja structure cyclique, et un ou plusieurs substituants R₃ identiques ou différents, occupant des positions distinctes quelconques sur le cycle, l'un au moins représentant un atome d'halogène, un groupe -OH, un groupe -CF₃, un radical aryle, un radical alkyle ou alkoxy en C1 à C5, un groupe -NO₂, le ou les autres substituants présentant l'une de ces significations ou un atome d'hydrogène, les sommets carbonés du peroxyde cyclique pouvant être le cas échéant substitués par un ou plusieurs substituants tels que définis pour R₃, deux substituants adjacents pouvant former une structure cyclique à 5 ou 6 chaînons, saturée ou insaturée, le cas échéant substituée par un ou plusieurs substituants R₃ en position quelconque, l'autre substituant Rₓ ou R_{y} pouvant être R₃,
et leurs sels d'addition avec des acides pharmacologiquement acceptables.

2. Molécules selon la revendication 1, **caractérisée en ce que** A représente un hétérocycle azoté choisi parmi une aminoquinoléine de formule (II) ou une 1,5-naphtyridine de formule (III) suivantes 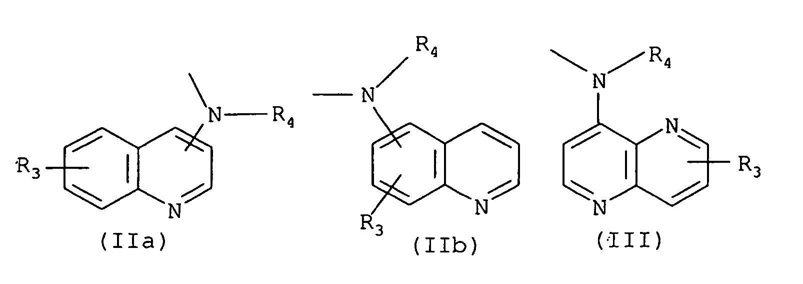 dans lesquelles
- R₃ représente, un ou plusieurs substituants identiques ou différents, occupant des positions distinctes, l'un au moins représentant un atome d'halogène, un groupe -OH, un groupe -CF₃, un radical aryle, un radical alkyle ou alkoxy en C1 à C5, un groupe -NO₂, le ou les autres substituants présentant l'une de ces significations ou un atome d'hydrogène,
- R₄ représente un radical alkyle en C1 à C5, linéaire, ramifié ou cyclique, ou un atome d'hydrogène.

3. Molécules selon la revendication 1, **caractérisées en ce que** A représente un radical de formule (IV)
R₅-CHOH- (IV)
dans laquelle R₅ représente un radical aryle ou un résidu hétérocyclique azoté.

4. Molécules selon la revendication 3, **caractérisées en ce que** R₅ est un radical 9-phénanthrényle ou 4-quinoléinyle, éventuellement substitué par un ou plusieurs groupes R₃.

5. Molécules selon la revendication 1, **caractérisées en ce que** A représente un résidu phénol-2 (aminométhyl) de formule (V) 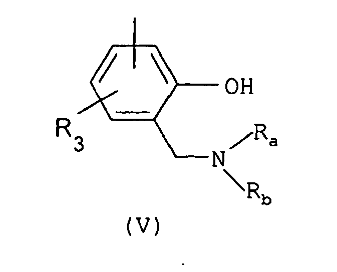 dans laquelle R₃, Rₐ et R_{b} sont tels que définis dans la revendication 1.

6. Molécules selon la revendication 1, caratérisée en ce que A représente un résidu biguanide choisi parmi les dérivés de proguanil de formule (VI) 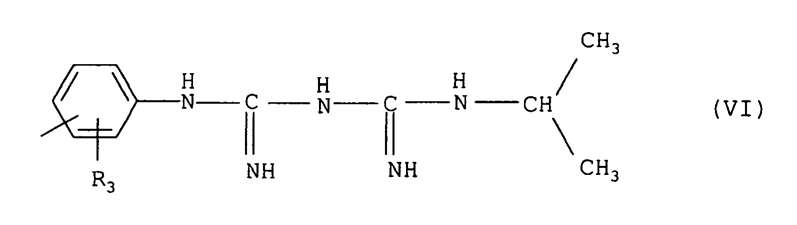 ou de cycloguanil de formule (VII) 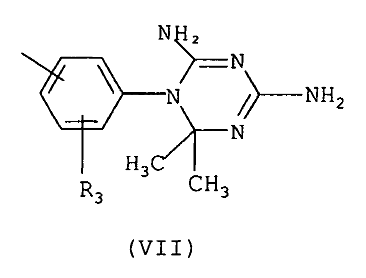 dans lesquelles R₃ est tel que défini dans la revendication 1.

7. Molécules selon la revendication 1, **caractérisées en ce que** A représente un résidu de pyrimidine et plus particulièrement de pyriméthamine de formule (VIII) 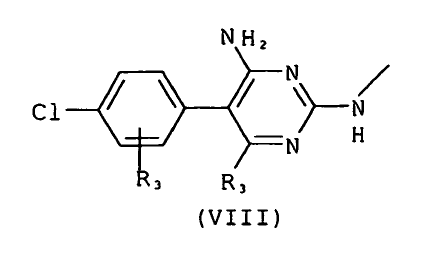 ou de formule (IX) 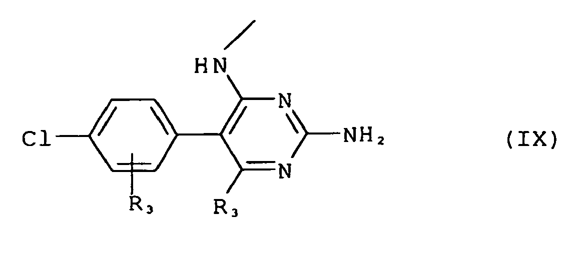 dans laquelle R₃ est tel que défini dans la revendication 1

8. Molécules selon la revendication 1 , **caractérisées en ce que** A représente un résidu acridine de formule (X) 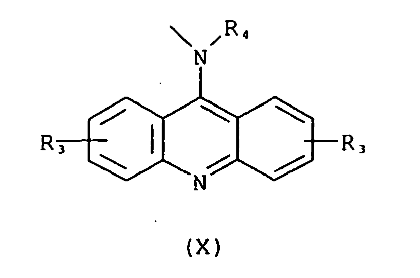 dans laquelle R₃ et R₄ sont tels que définis, respectivement, dans les revendications let 2.

9. Molécules selon l'une quelconque des revendications 1 à 8, **caractérisées en ce que** Rₓ et R_{y} forment ensemble un peroxyde cyclique.

10. Molécules selon la revendication 9, **caractérisées en ce que** Rₓ et R_{y} représentent un trioxane substitué par un ou plusieurs substituants R₃.

11. Molécules selon l'une quelconque des revendications 1 à 10, **caractérisées en ce que** R₃ représente un seul substituant, ce substituant étant un atome d'halogène choisi parmi F, Cl, Br, I, ou 2 substituants occupant des positions distinctes, l'un représentant un atome d'halogène choisi parmi F, Cl, Br, I, l'autre un groupe alcoxy.

12. Molécules selon l'une quelconque des revendications 1 à 11, caratérisées en ce que Z₁ et Z₂ représentent un radical cyclohexyle ou bis-cyclopentyle.

13. Procédé de préparation de molécules duales selon la revendication 1 dans laquelle A est une amino-quinoléine et Rₓ et R_{y} forment un trioxane, **caractérisé par**
a) la réaction d'un composé de formule (XI) 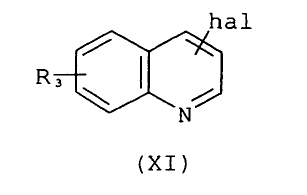 dans laquelle R₃ est tel que défini ci-dessus, et "hal" représente un atome d'halogène, avec une dérivé diaminé de formule (XII)
R₄-NH-Y₁-U₁ (XII)
où R₄ et Y₁ sont tels que définis ci-desus et U₁ représente un groupe -NH₂,
ce qui conduit à l'obtention d'un composé de formule (XIII) 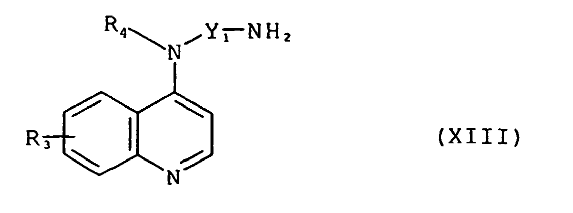 dans laquelle, R₃, R₄ et Y₁ sont tels que définis ci-dessus,
b) - l'irradiation en présence d'oxygène moléculaire et d'un photosensibilisateur, d'un dérivé de formule (XIV) à (XVII) suivante 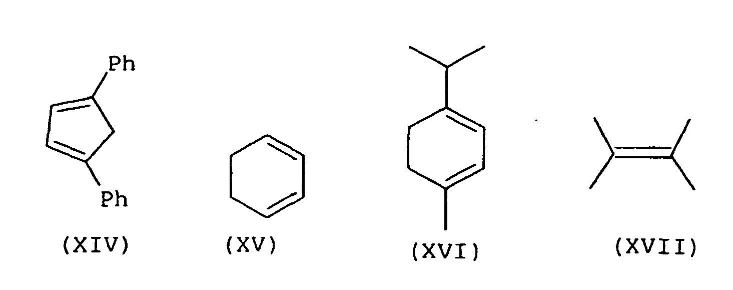 suivie de la réaction avec une dicétone, telle que la 1,4-cyclohexadione de formule (XVIII) ou la *cis*-bicyclo[3.3.0]octane-3,7-dione de formule (XIX) 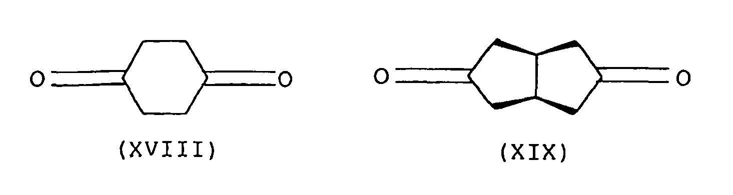 conduisent à des trioxanes fonctionnalisés par une cétone, de formule générale (XX) 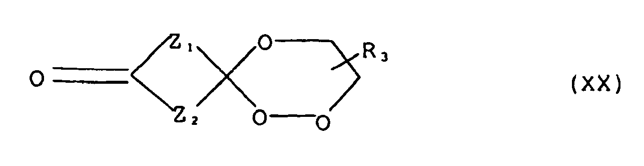 dans laquelle Z₁, Z₂ et R₃ sont tels que définis ci-dessus,
c) - le couplage du dérivé de formule (XIII) avec le trioxane de formule (XX), par amination réductrice, suivi le cas échéant d'une réaction avec un acide pharmaceutiquement acceptable, pour obtenir le produit de couplage sous forme de sel.

14. Compositions phamaceutiques, **caractérisées en ce qu'**elles renferment une quantité efficace d'au moins un produit de couplage tel que défini dans l'une quelconque des revendications 1 à 12, en association avec un véhicule phamaceutiquement inerte.

15. Compositions pharmaceutiques selon la revendication 14, administrables par voie orale, rectale ou injectable.

16. Compositions selon la revendication 15, **caractérisées en ce qu'**elles renferment de 10 à 100 mg de principe actif par unité de prise en vue d'une administration par voie orale.

17. Compositions selon la revendication 16, **caractérisées en ce que**, pour l'administration par voie orale, elles se présentent sous forme de comprimés, pilules, tablettes, gélules, gouttes.

18. Compositions selon la revendication 15, **caractérisées en ce qu'**elles renferment par unité de prise de 10 à 50 mg de principe actif en vue d'une administration par voie injectable.

19. Compositions selon la revendication 15, **caractérisées en ce que**, pour une administration par voie injectable, elles se présentent sous forme de solutions injectables par voie intraveineuse, sous cutanée ou intramusculaire, élaborées à partir de solutions stériles ou stérilisables, ou encore de suspensions ou d'émulsions.

20. Compositions pharmaceutiques selon la revendication 14 ou 15 destinées au traitement du paludisme.

21. Utilisation des molécules duales selon l'une quelconque de revendications 1 à 12 pour l'élaboration de médicaments à activité antipaludique.

## Claims

1. Dual molecules **characterized in that** they are coupling products having formula (I) 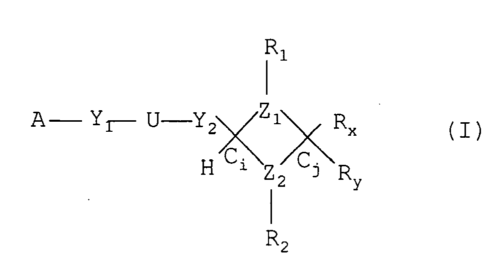 wherein
- A represents a residue of a molecule with anti-malarial activity, of the group comprising,
- a nitrogeneous heterocycle, selected amongst an aminoquinoline of following formula (II) or a 1,5-naphtypyridine of following formula (III) 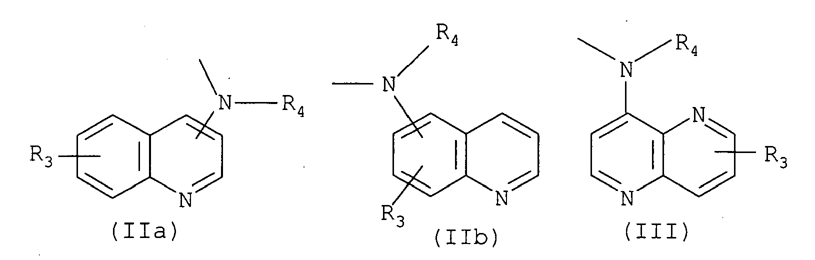
Wherein
- R₃ represents one or more identical or different substituents, occupying separate positions, at least one representing an halogen atom, an -OH group, a -CF₃ group, an aryl radical, a C₁-C₅ alkyl or alkoxy radical, a -NO₂ group, the other substituent or substituents having one of these meanings or an hydrogen atom;
- R₄ represents a linear, branched or cyclic C1-C5 alkyl radical, or a hydrogen atom;
- a radical of formula (IV) R₅-CHOH- wherein R₅ represents an aryl radical or a nitrogenous heterocycle,
- a phenol-2 (aminomethyl) residue of formula (V) 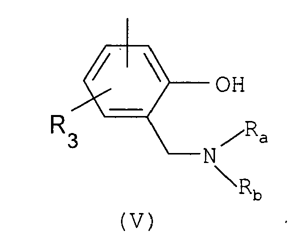
wherein R₃ is such as above defined, and Ra and Rb, identical or different, represent an hydrogen atom or a C1 to C5 alkyl radical,
- a biguanide residue selected from proguanil derivatives of formula (VI) 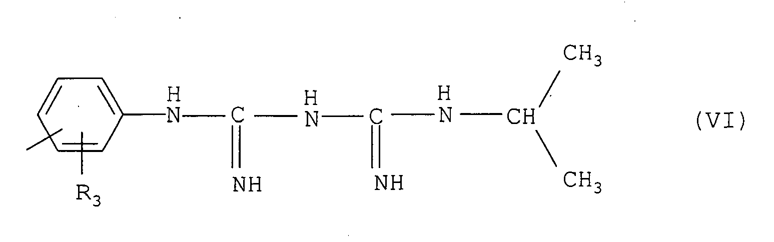
or cycloguanil of formula (VII) 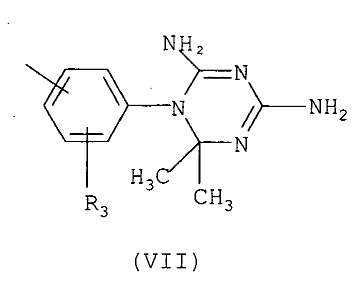 wherein R₃ is as above defined,
- a pyrimidine residue and more particularly of pyrimethamine according to formula (VIII) 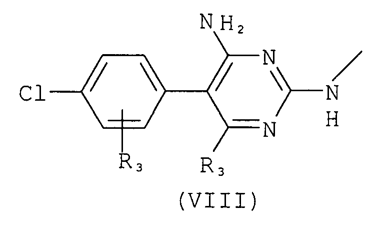
or formula (IX) 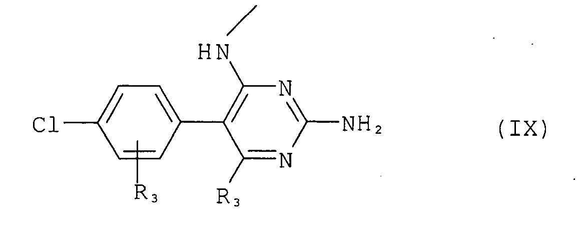 wherein R₃ is as defined above,
- an acridine residue of formula (X) 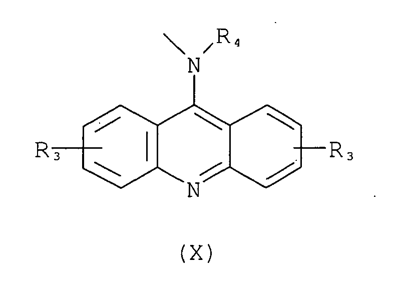
wherein R₃ and R₄ are as above defined,
- Y₁ and Y₂, identical or different, represent a linear or branched C1 to C5 alkylene chain, optionally containing one or more amine, amide, sulfonamide, carboxyl, hydroxyl, ether or thioether radicals, the C1-C5 alkylene chain being optionally substituted by a C1-C5 alkyl radical, with the possibility of either Y₁ or Y₂ being absent,
- U is an amine, amide, sulfonamide, carboxyl, ether or thioether function, said function linking Y₁ and Y₂,
- Z₁ and Z₂, identical or different, represent a saturated or unsaturated, linear, branched or cyclic arylene or alkylene radical, with the possibility of either Z₁ or Z₂ being absent, or Z₁ + Z₂ together represent a polycyclic structure including the junction carbons Ci and Cj,
- R₁ and R₂, identical or different, represent an hydrogen atom or a functional group capable of increasing the hydrosolubility of the dual molecule, advantageously selected from -COOH, -OH, -N(Rₐ, R_{b}) where Rₐ and R_{b}, are as above defined;
- Rₓ and R_{y} form a cyclic peroxide with 4 to 8 links, which may comprise 1 or 2 additional oxygen atoms in the cyclic structure, Cj being one of the peaks of this cyclic peroxide, or
- Rₓ or R_{y} is a cyclic peroxide with 4 to 8 chains, which may comprise 1 or 2 additional oxygen atoms in the cyclic structure, and one or several R3 substituents occupying any separate positions on the cycle, at least one representing an halogen atom, an - OH group, a -CF₃ group, an aryl radical, a C1-C5 alkyl or alkoxy radical, a -NO₂ group, the other substituent or substituents having one of these meanings or an hydrogen atom, the carbon peaks of the cyclic peroxide being optionally substituted by one or several substituents such as defined for R₃, two adjacent substituents optionally forming a saturated or unsaturated cyclic structure with 5 or 6 links, optionally substituted by one or several R₃ substituents at any position, the other substituent Rₓ or R_{y} optionally being R₃,
and their addition salts with pharmacologically acceptable acids.

2. Molecules according to claim 1, wherein a represents a nitrogenous heterocycle selected from an aminoquinoline of following formula (II) or a 1,5-naphtypyridine of following formula (III) 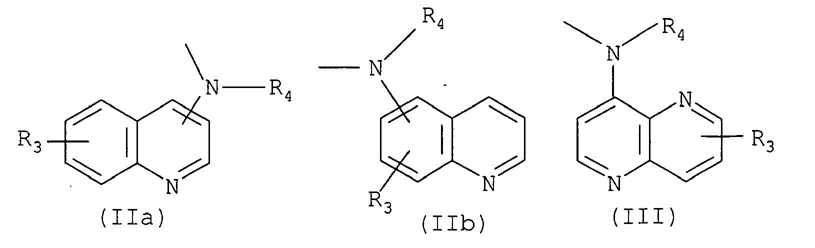 wherein
- R₃ represents one or more, identical or different, substituents occupying separate positions, at least one representing an halogen atom, an -OH group, a -CF₃ group, an aryl radical, a C1-C5 alkyl or alkoxy radical, a -NO₂ group, the other substituent or substituents having one of these meanings or an hydrogen atom,
- R₄ represents a linear, branched or cyclic C1-C5 alkyl radical, or an hydrogen atom.

3. Molecules according to claim 1, wherein A represents a radical of formula (IV)
R₅-CHOH- (IV)
wherein R₅ represents an aryl radical or a nitrogenous heterocyclic residue.

4. Molecules according to claim 3, wherein R₃ is a 9-phenanthrenyl or 4-quinolinyl radical, optionally substituted by one or more R₃ groups.

5. Molecules according to claim 1, wherein A represents a phenol-2(aminomethyl) residue of formula (V) 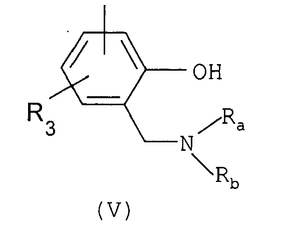 wherein R₃, Rₐ and R_{b} are such as defined in claim 1.

6. Molecules according to claim 1, wherein A represents a biguanide residue selected from proguanil derivatives of formula (VI) 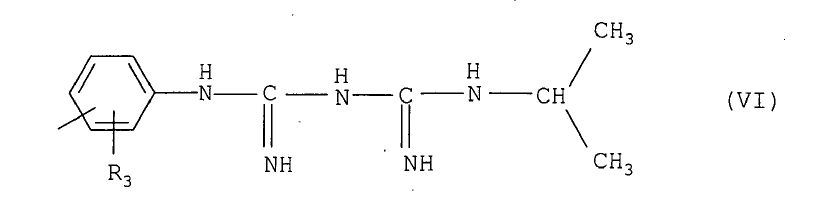 or cycloguanil of formula (VII) 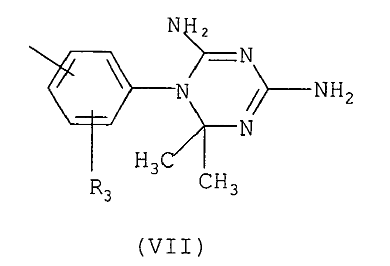 wherein R₃ is as in claim 1.

7. Molecules according to claim 1, wherein A represents a pyrimidine residue and more particularly of pyrimethamine of formula (VIII) 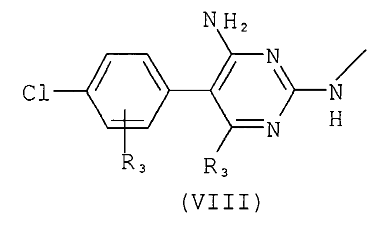 or formula (IX) 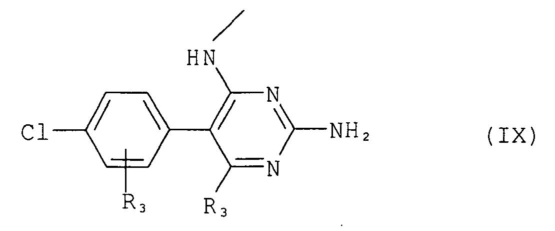 wherein R₃ is as defined in claim 1.

8. Molecules according to claim 1, wherein A represents an acridine residue of formula (X) 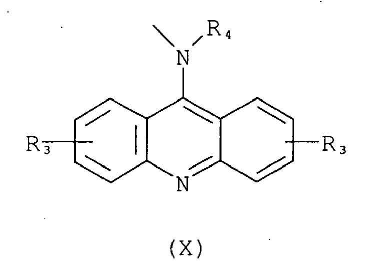 wherein R₃ and R₄ are such as defined in claims 1 and 2, respectively.

9. Molecules according to any of claims 1 to 8, wherein Rₓ and R_{y} form a cyclic peroxide together.

10. Molecules according to claim 9, wherein Rₓ and R_{y} represent a trioxane substituted by one or more substituents R₃.

11. Molecules according to anyone of claims 1 to 10, wherein R₃ represents only one substituent, said substituent being an halogen atom selected from F, Cl, Br, I or 2 substituents occupying separate positions, one representing an halogen atom selected from F, Cl, Br, I, and the other one an alcoxy group.

12. Molecules according to anyone of claims 1 to 11, wherein Z₁ and Z₂ represent a cyclohexyl or bi-cyclopentyl radical.

13. A method to preparing dual molecules according to claim 1 wherein A is an amino-quinoline and Rₓ and R_{y} form a trioxane, comprising
a)-reacting a compound of formula (XI) 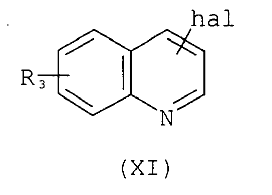 wherein R₃ is such as above defined and "hal" represents an halogen atom, with a diamine derivative according to formula (XII)
R₄-NH-Y₁-U₁ (XII)
where R₄ and Y₁ are as above defined and U₁ represents a -NH₂ group, producing a compound according to formula (XIII) 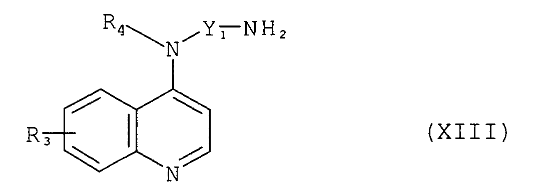 wherein R₃, R₄ and Y₁ are such as above defined,
b)-irradiating in the presence of molecular oxygen and a photosensitising agent, of a derivative according to formulae (XIV) to (XVII) below 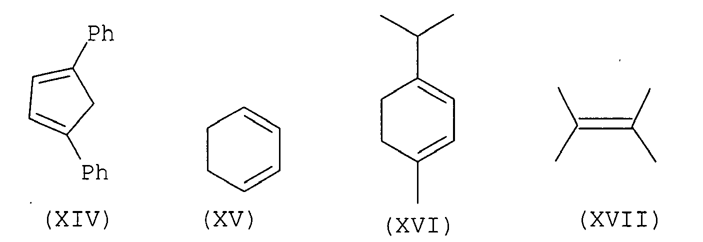 followed by the reaction with a diketone, such as 1,4-cyclohexadione according to formula (XVIII) or *cis-*bicyclo(3.3.0]octane-3,7-dione of formula (XIX) 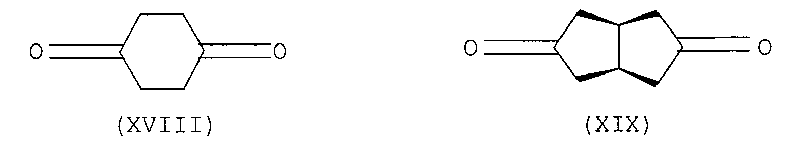 producing trioxanes functionalised with a ketone, of general formula (XX) 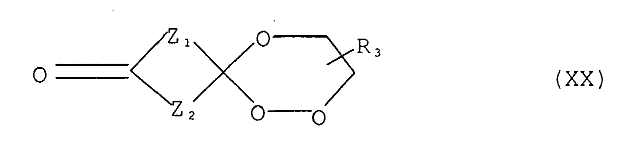 wherein Z₁, Z₂ and R₃ are as defined above,
c)-coupling of the derivative according to formula (XIII) with the trioxane of formula (XX), by reductive amination, optionally followed by a reaction with a pharmaceutically acceptable acid, to obtain the coupling product in salt form.

14. Pharmaceutical compositions, comprising an effective quantity of at least one coupling product such as defined in any one of claims 1 to 12, in association with a pharmaceutically inert vehicle.

15. Pharmaceutical compositions according to claim 14, which may be administered by the oral, rectal or injectable route.

16. Compositions according to claim 15, comprising 10 to 100 mg of active principle per dosage unit for oral administration.

17. Compositions according to claim 16, wherein, for oral administration, they are presented in the form of tablets, pills, lozenges, capsules or drops.

18. Compositions according to claim 15, comprising 10 to 50 mg of active ingredient per dosage unit for administration by injection.

19. Compositions according to claim 15, wherein for administration by injection, they are presented in the form of solutions for injection by the intravenous, subcutaneous or intramuscular route, produced from sterile or sterilisable solutions, or suspensions or emulsions.

20. Pharmaceutical compositions, according to claims 14 or 15 intended for the treatment of malaria.

21. The use of dual molecules according to any one of claims 1 to 12 for making drugs having an anti-malarial activity.

## Patentansprüche

1. Doppelmoleküle, **dadurch gekennzeichnet, dass** es sich um Kopplungsprodukte handelt, die der Formel (I) 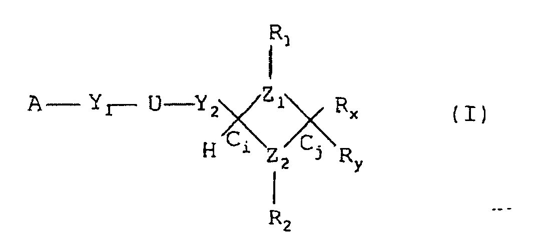 entsprechen, wobei
- A einen Molekülrest mit einer Antimalariawirkung der Gruppe darstellt, die Folgendes umfasst:
- einen stickstoffhaltigen Heterocyclus, der aus einem Aminochinolin der folgenden Formel (II) oder einem 1,5-Naphthyridin der folgenden Formel (III) ausgewählt ist, 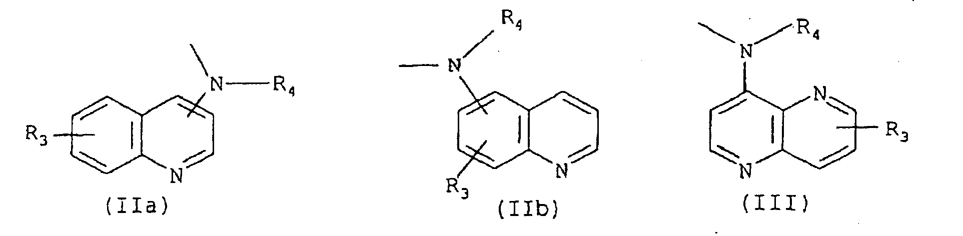 wobei
- R₃ einen oder mehrere identische oder verschiedene Substituenten darstellt, die verschiedene Positionen einnehmen, wobei mindestens einer ein Halogenatom, eine -OH-Gruppe, eine -CF₃-Gruppe, einen Arylrest, einen C1- bis C5-Alkyl- oder Alkoxyrest, eine -NO₂-Gruppe darstellt und der oder die anderen Substituenten eine dieser Bedeutungen haben oder ein Wasserstoffatom darstellen,
- R₄ einen linearen, verzweigten oder cyclischen C1- bis C5-Alkylrest oder ein Wasserstoffatom darstellt,
- einen Rest der Formel (IV) R₅-CHOH, wobei R₅ einen Arylrest oder einen stickstoffhaltigen Heterocyclus darstellt,
- einen Phenol-2(aminomethyl)-Rest der Formel (V) 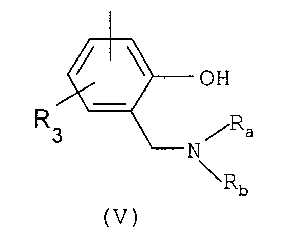 wobei R₃ oben definiert ist, und Rₐ und R_{b}, die gleich oder verschieden sind, ein Wasserstoffatom oder einen C1- bis C5-Rest darstellen,
- einen Biguanidrest, der aus den Proguanilderivaten der Formel (VI) 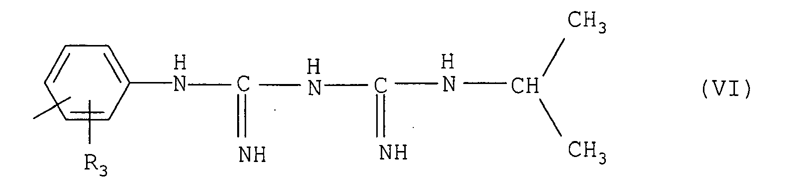 oder dem Cycloguanil der Formel (VII), 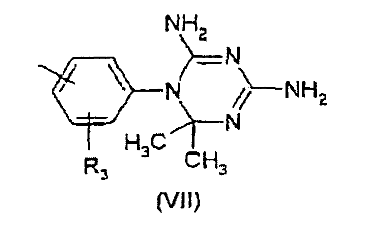 wobei R₃ oben definiert ist, ausgewählt ist,
- einen Pyrimidinrest und insbesondere ein Pyrimethamin der Formel 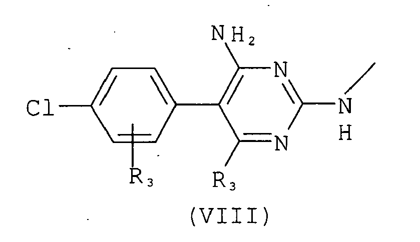 oder der Formel (IX) 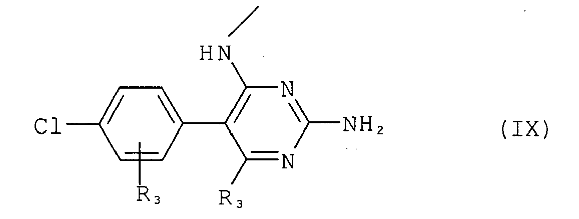 wobei R₃ wie oben definiert ist,
- einen Pyrimidinrest,
- einen Acridinrest der Formel (X) 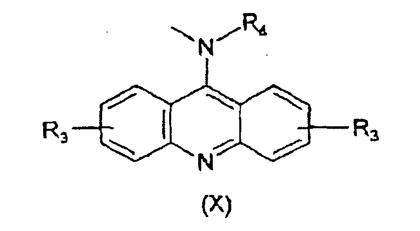 wobei R₃ und R₄ oben definiert sind,
- Y₁ und Y₂, die gleich oder verschieden sind, eine lineare oder verzweigte C1- bis C5-Alkylenkette darstellen, die gegebenenfalls einen oder mehrere Amin-, Amid- Sulfonamid-, Carboxyl-, Hydroxyl-, Ether- oder Thioetherreste enthält, wobei diese C1- bis C5-Alkylenkette gegebenenfalls durch einen C1- bis C5-Alkylrest substituiert sein kann, wobei entweder Y₁ oder Y₂ fehlen kann,
- U eine Amin-, Amid-, Sulfonamid-, Carboxyl-, Ether- oder Thioetherfunktion sein kann, wobei diese Funktion die Verbindung zwischen Y₁ und Y₂ gewährleistet,
- Z₁ und Z₂, die gleich oder verschieden sind, einen Arylen- oder einen linearen, verzweigten oder cyclischen, gesättigten oder ungesättigten, Alkylenrest darstellen, wobei entweder Z₁ oder Z₂ fehlen kann oder die Gesamtheit aus Z₁ + Z₂ eine polycyclische Struktur darstellt, die die Verbindungskohlenstoffe Ci und Cj einschließt,
- R₁ und R₂, die gleich oder verschieden sind, ein Wasserstoffatom oder eine funktionelle Gruppe darstellen, die dazu fähig ist, die Wasserlöslichkeit des Doppelmoleküls zu erhöhen, und vorzugsweise aus -COOH, -OH, -N(Rₐ, R_{b}) ausgewählt sind, wobei Rₐ und R_{b} oben definiert sind,
- Rₓ und R_{y} ein cyclisches Peroxid mit 4 bis 8 Gliedern bilden, das 1 oder 2 zusätzliche Sauerstoffatome in der cyclischen Struktur umfassen kann, wobei Cj einer der Eckpunkte des cyclischen Peroxids ist, oder
- Rₓ oder R_{y} ein cyclisches Peroxid mit 4 bis 8 Gliedern darstellt, das in der cyclischen Struktur 1 oder 2 zusätzliche Sauerstoffatome oder einen oder mehrere identische oder verschiedene Substituenten R₃ umfassen kann, die im Ring beliebige verschiedene Positionen einnehmen, wobei mindestens eines davon ein Halogenatom, eine - OH-Gruppe, eine -CF₃-Gruppe, einen Arylrest, einen C1- bis C5-Alkyl- oder Alkoxyrest, eine -NO₂-Gruppe darstellt, wobei der oder die anderen Substituenten eine dieser Bedeutungen hat oder ein Wasserstoffatom darstellt, die Kohlenstoff-Eckpunkte des cyclischen Peroxids gegebenenfalls durch einen oder mehrere durch R₃ definierten Substituenten substituiert sein können, zwei benachbarte Substituenten eine cyclische, gesättigte oder ungesättigte Struktur mit 5 oder 6 Gliedern bilden können, die gegebenenfalls durch ein oder mehrere Substituenten R₃ in einer beliebigen Position substituiert sein kann, wobei der andere Substituent, Rₓ oder R_{y}, R₃ sein kann,
und deren Additionssalze mit pharmakologisch annehmbaren Säuren.

2. Moleküle nach Anspruch 1, **dadurch gekennzeichnet, dass** A einen stickstoffhaltigen Heterocyclus darstellt, der ausgewählt ist aus einem Aminochinolin der folgenden Formel (II) oder einem 1,5-Naphthyridin der folgenden Formel (III) 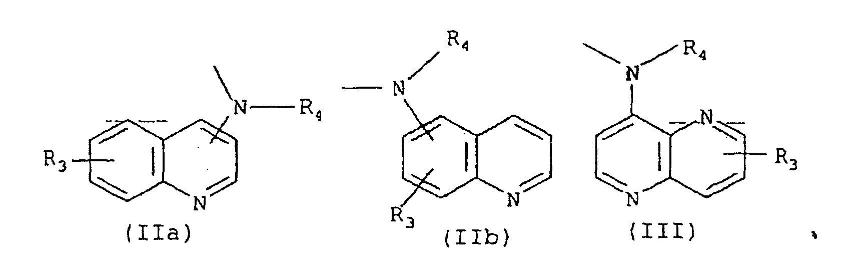 wobei
- R₃ einen oder mehrere identische oder verschiedene Substituenten darstellt, die beliebige Positionen einnehmen, wobei mindestens einer davon ein Halogenatom, eine -OH-Gruppe, eine -CF₃-Gruppe, einen Arylrest, einen C1- bis C5-Alkyl- oder Alkoxyrest, eine -NO₂-Gruppe darstellt, wobei der oder die anderen Substituenten eine dieser Bedeutungen haben oder ein Wasserstoffatom sind,
- R₄ einen linearen, verzweigten oder cyclischen C1- bis C5-Alkylrest oder ein Wasserstoffatom darstellt.

3. Moleküle nach Anspruch 1, **dadurch gekennzeichnet, dass** A einen Rest der Formel (IV)
R₅-CHOH- (IV)
veranschaulicht, wobei R₅ einen Arylrest oder einen stickstoffhaltigen, heterocyclischen Rest darstellt.

4. Moleküle nach Anspruch 3, **dadurch gekennzeichnet, dass** R₅ ein 9-Phenanthrenyl- oder 4-Chinolinylrest ist, der gegebenenfalls durch eine oder mehrere R₃-Gruppen substituiert ist.

5. Moleküle nach Anspruch 1, **dadurch gekennzeichnet, dass** A einen Phenol-2-(aminomethyl)-Rest der Formel (V) 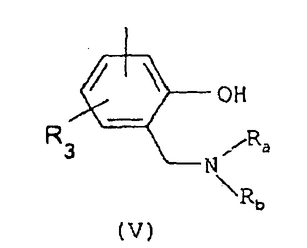 darstellt, in dem R₃, Rₐ und R_{b} wie in Anspruch 1 definiert sind.

6. Moleküle nach Anspruch 1, **dadurch gekennzeichnet, dass** A einen Biguanidrest darstellt, der aus den Proguanilderivaten der Formel (VI) 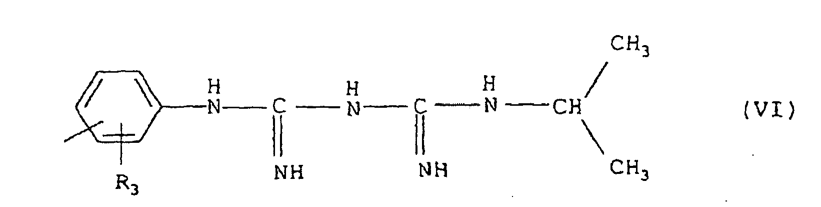 oder den Cycloguanilderivaten der Formel (VII) 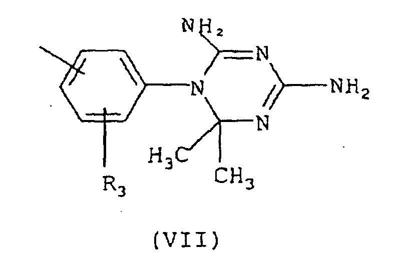 ausgewählt ist, wobei R₃ wie in Anspruch 1 definiert ist.

7. Moleküle nach Anspruch 1, **dadurch gekennzeichnet, dass** A einen Pyrimidinrest und insbesondere das Pyrimethamin der Formel (VIII) 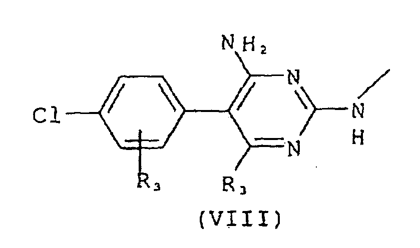 oder der Formel (IX) 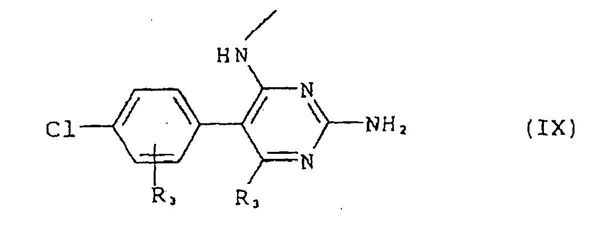 darstellt, wobei R₃ wie in Anspruch 1 definiert ist.

8. Moleküle nach Anspruch 1, **dadurch gekennzeichnet, dass** A einen Acridinrest der Formel (X) 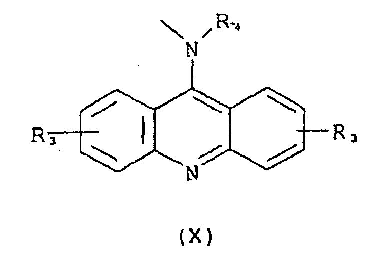 darstellt, wobei R₃ und R₄ jeweils wie in den Ansprüchen 1 und 2 definiert sind.

9. Moleküle nach einem der Ansprüche 1 bis 8, **dadurch gekennzeichnet, dass** Rₓ und R_{y} zusammen ein cyclisches Peroxid bilden.

10. Moleküle nach Anspruch 9, **dadurch gekennzeichnet, dass** Rₓ und R_{y} ein Trioxan darstellen, das durch einen oder mehrere Substituenten R₃ substituiert ist.

11. Moleküle nach einem der Ansprüche 1 bis 10, **dadurch gekennzeichnet, dass** R₃ einen einzigen Substituenten darstellt, wobei dieser Substituent ein aus F, Cl, Br, I ausgewähltes Halogenatom ist, oder 2 Substituenten darstellt, die beliebige Positionen einnehmen, wobei einer davon ein aus F, Cl, Br, I ausgewähltes Halogenatom und der andere eine Alkoxygruppe darstellt.

12. Moleküle nach einem der Ansprüche 1 bis 11, **dadurch gekennzeichnet, dass** Z₁ und Z₂ einen Cyclohexyl- oder Biscyclopentylrest darstellen.

13. Verfahren zur Herstellung von Doppelmolekülen nach Anspruch 1, wobei A ein Aminochinolin ist und Rₓ und R_{y} ein Trioxan bilden, **gekennzeichnet durch**
a) die Umsetzung einer Verbindung der Formel (XI) 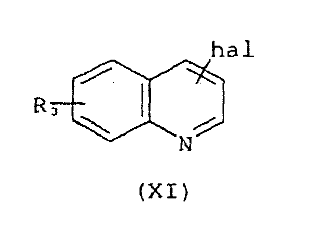 wobei R₃ wie oben definiert ist und "hal" ein Halogenatom darstellt, mit einem Diaminderivat der Formel (XII)
R₄-NH-Y₁-U₁ (XII)
wobei R₄ und Y₁ wie oben definiert sind und U₁ eine -NH₂-Gruppe darstellt, die zum Erhalt einer Verbindung der Formel (XIII) 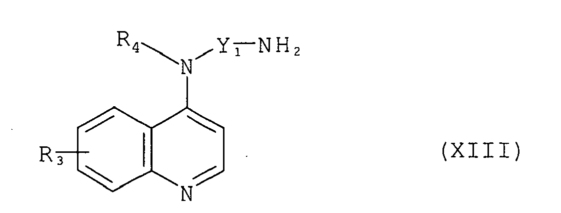 führt, wobei R₃, R₄ und Y₁ wie oben definiert sind,
b) die Bestrahlung in Gegenwart von molekularem Sauerstoff und einem Photosensibilisator eines Derivats der folgenden Formel (XIV) bis (XVII), 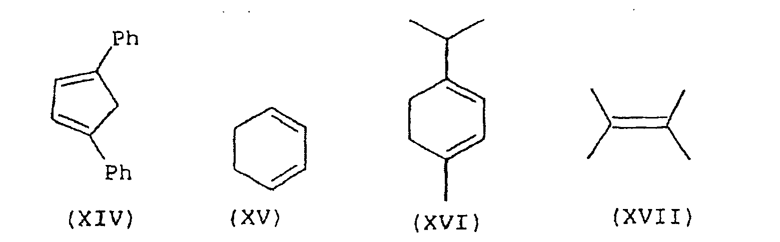 gefolgt von der Reaktion mit einem Diketon wie dem 1,4-Cyclohexadion der Formel (XVIII) oder dem cis-Bicyclo[3.3.0]octan-3,7-dion der Formel (XIX), 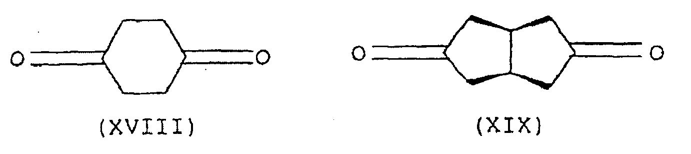 die zu ketonfunktionalisierten Trioxanen der allgemeinen Formel (XX) 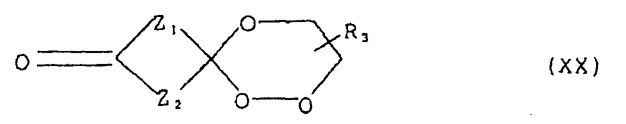 führt, wobei Z₁, Z₂ und R₃ wie oben definiert sind,
c) die Kopplung des Derivats der Formel (XIII) mit dem Trioxan der Formel (XX) **durch** eine reduktive Aminierung, gegebenenfalls gefolgt von einer Umsetzung mit einer pharmazeutisch annehmbaren Säure, wodurch das Kupplungsprodukt in Form eines Salzes erhalten wird.

14. Pharmazeutische Zusammensetzungen, **dadurch gekennzeichnet, dass** sie eine wirksame Menge mindestens eines Kopplungsprodukts wie demjenigen, das in einem der Ansprüche 1 bis 12 definiert ist, zusammen mit einem inerten pharmazeutischen Hilfsmittel enthalten.

15. Pharmazeutische Zusammensetzungen nach Anspruch 14, die oral, rektal oder injizierbar verabreichbar sind.

16. Zusammensetzungen nach Anspruch 15, **dadurch gekennzeichnet, dass** sie mit Hinblick auf eine orale Verabreichung 10 bis 100 mg des Wirkprinzips pro Dosiseinheit enthalten.

17. Zusammensetzungen nach Anspruch 16, **dadurch gekennzeichnet, dass** zur oralen Verabreichung in Form von Tabletten, Pillen, Pastillen, Kapseln, Tropfen vorliegen.

18. Zusammensetzungen nach Anspruch 15, **dadurch gekennzeichnet, dass** sie mit Hinblick auf eine injizierbare Verabreichung pro Dosiseinheit 10 bis 50 mg des Wirkprinzips enthalten.

19. Zusammensetzungen nach Anspruch 15, **dadurch gekennzeichnet, dass** sie zur injizierbaren Verabreichung in Form von Lösungen vorliegen, die intravenös, subkutan oder intramuskulär injizierbar sind, aus sterilen oder sterilisierbaren Lösungen oder darüber hinaus aus Suspensionen oder Emulsionen hergestellt werden.

20. Pharmazeutische Zusammensetzungen nach Anspruch 14 oder 15, die zur Behandlung von Malaria bestimmt sind.

21. Verwendung von Doppelmolekülen nach einem der Ansprüche 1 bis 12 zur Herstellung von Medikamenten mit Antimalariawirkung.
